(19) Europäisches Patentamt

European Patent Office

Office européen des brevets

(11) **EP 2 380 626 B1**

(12) **EUROPEAN PATENT SPECIFICATION**

(45) Date of publication and mention
of the grant of the patent:
**04.03.2015 Bulletin 2015/10**

(21) Application number: **11075091.6**

(22) Date of filing: **30.09.2005**

(51) Int Cl.:
*A61B 5/0464* (2006.01)   *A61B 5/00* (2006.01)
*A61B 5/046* (2006.01)   *A61B 5/0452* (2006.01)
*A61N 1/362* (2006.01)

(54) **Arrhythmia classification and therapy selection**

Arrhythmie-Einstufung und Therapieauswahl

Classification d'arythmie et sélection de thérapie

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **30.09.2004 US 955831
23.11.2004 US 995655
23.11.2004 US 995704
23.11.2004 US 996340**

(43) Date of publication of application:
**26.10.2011 Bulletin 2011/43**

(62) Document number(s) of the earlier application(s) in
accordance with Art. 76 EPC:
**05800765.9 / 1 804 913**

(73) Proprietor: **Cardiac Pacemakers, Inc.
St. Paul, Minnesota 55112 (US)**

(72) Inventors:
• **Kim, Jaeho
Redmond
Washington 98052 (US)**
• **Bocek, Joseph
Seattle
Washington 98102 (US)**
• **Lovett, Eric G.
Mendota Heights
Minnesota 55120 (US)**
• **Cazares, Shelley Marie
Washington DC 20001 (US)**
• **Lin, Yayun
St. Paul
Minnesota 55113 (US)**
• **Sathaye, Alok
New York
NY 10011 (US)**

(74) Representative: **Pfenning, Meinig & Partner GbR
Patent- und Rechtsanwälte
Joachimstaler Strasse 12
10719 Berlin (DE)**

(56) References cited:
**US-A- 4 880 005      US-A- 5 251 621
US-A1- 2004 111 120      US-A1- 2004 167 579
US-A1- 2004 171 959      US-B1- 6 438 407**

EP 2 380 626 B1

**Description**

**FIELD OF THE INVENTION**

The present invention relates generally to implantable medical devices, and more particularly, to classifying cardiac rhythms and providing arrhythmia therapy.

**BACKGROUND OF THE INVENTION**

Rhythmic contractions of a healthy heart are normally initiated by the sinoatrial (SA) node, specialized cells located in the upper right atrium. The SA node is the normal pacemaker of the heart. When functioning normally, the heart produces rhythmic contractions and is capable of pumping blood throughout the body. However, due to disease or injury, the heart rhythm may become irregular resulting in diminished blood circulation. Arrhythmia is a general term used to describe heart rhythm irregularities arising from a variety of physical conditions and disease processes.

A cardiac tachyarrhythmia that originates in a non-ventricular region of the heart is denoted a supra-ventricular tachyarrhythmia (SVT). Atrial fibrillation and atrial flutter are examples of SVT. Both conditions are characterized by rapid contractions of the atria resulting in hemodynamically inefficient pumping action.

Cardiac arrhythmias originating in a ventricular region of the heart are denoted ventricular tachyarrhythmias (VT). Some types of ventricular tachyarrhythmia are characterized by rapid ventricular contractions that are fairly regular and coordinated. Such rhythms can degenerate into ventricular fibrillation (VF). Ventricular fibrillation produces extremely rapid, non-coordinated contractions of the ventricles and is fatal unless the heart is returned to sinus rhythm within a few minutes.

Implantable cardiac rhythm management (CRM) devices, including pacemakers and implantable cardioverter/defibrillators (ICDs), have been used to deliver effective treatment to patients with serious cardiac arrhythmias. Leads extending into the patient's heart are connected to electrodes electrically coupled to the myocardium for sensing the heart's electrical signals and for delivering stimulation pulses to the heart in accordance with various therapies for treating the arrhythmias.

A number of CRM devices having various modes for sensing and pacing one or more heart chambers and can treat cardiac arrhythmias using a variety of tiered therapies. These tiered therapies range from the delivery of low energy pacing pulses timed to assist the heart in maintaining pumping efficiency to high energy shocks to terminate fibrillation. To effectively deliver these treatments the CRM device must first identify the type of arrhythmia that is occurring.

For the reasons stated above, and for other reasons stated below which will become apparent to those skilled in the art upon reading the present specification, there is a need in the art for methods and systems that accurately identify and treat cardiac arrhythmias. There exists a further need to deliver proper cardiac therapy. The present invention fulfills these and other needs.

US 2004/0167579 describes an implantable medical device according to the first past of claim 1, for providing therapy in response to detected events. On detection of a tachycardia episode, the device determines the morphology of the tachycardia, and compares it with one or morphology templates stored in memory to see if it is similar to the morphology of at least one previous episode. When there is a morphology match, the device selects the hierarchy of therapies that is associated with the matching template and applies therapies according to the selected hierarchy. US 2004/0111120 describes methods and apparatus for discriminating polymorphic tachyarrhythmia from monomorphic tachyarrhythmias facilitating detection of defibrillation. US 2004/0171959 describes a device for determining termination of an identified tachyarrhythmia episode. US-A-4880 005 discloses a heat pacemaker that detects pace-terminable tadrycardia conditions in accordance with sustained high rate criticia.

**SUMMARY OF THE INVENTION**

The invention provides a medical system according to claim 1. Preferred embodiments are defined in the claims dependent thereon. A more complete understanding of the invention, will become apparent and appreciated by referring to the following detailed description and claims taken in conjunction with the accompanying drawings.

**BRIEF DESCRIPTION OF THE DRAWINGS**

Figures 1A and 1B are flowcharts of methods for classifying cardiac rhythms in accordance with embodiments of the invention;
Figures 2A and 2B are flowcharts illustrating a process in which a degree of similarity is determined between a SVT template and a heart signal in accordance with embodiments of the invention;

Figure 2C is a flowchart that illustrates a process in which a template is generated for an arrhythmia morphology that does not require antitachyarrhythmia therapy in accordance with embodiments of the invention;

Figure 3 is a flow chart that illustrates a method in which a degree of similarity is determined between a monitored heart signal and a SVT template selected from a group of candidate SVT templates in accordance with embodiments of the invention;

Figure 4 is a flowchart that illustrates a method in which a heart signal is compared to a normal sinus rhythm and a SVT template in accordance with embodiments of the invention;

Figure 5 is a flow chart that illustrates methods by which a SVT template is discarded from a storage medium and a new SVT template is added in accordance with embodiments of the invention;

Figure 6 is a flow chart that illustrates an embodiment of a process in which a heart signal from a tachyarrhythmia episode is compared to a normal sinus rhythm (NSR) template and then compared to a supraventricular tachyarrhythmia (SVT) template in accordance with embodiments of the invention;.

Figure 7 is a block diagram of an implantable device configured to determine similarity between a heart signal and at least one SVT template and to suppress ventricular antitachyarrhythmia therapy based on the similarity in accordance with embodiments of the invention;

Figure 8 shows an example of a normal sinus rhythm morphology;

Figure 9 shows an example of a SVT morphology;

Figures 10 and 11 are flowcharts of methods for classifying cardiac rhythms and delivering therapy to a patient in accordance with embodiments of the invention;

Figure 12 is a partial view of an implantable medical device suitable for implementing arrhythmia classification and therapy delivery methods in accordance with embodiments of the invention;

Figures 13A and 13B are a block diagram of a cardiac rhythm management system in accordance with embodiments of the invention;

Figure 14 is a flowchart illustrating a method for classifying arrhythmia and delivering cardiac therapy to a patient in accordance with embodiments of the invention;

Figure 15 is a graph illustrating a cardiac signal having cardiac waveform features useful for template creation in accordance with embodiments of the invention;

Figures 16A and 16B are graphs illustrating the use of rate and shock channel signals for arrhythmia classification in accordance with embodiments of the invention;

Figure 17 is a flowchart illustrating a method of forming a template that may be used for arrhythmia classification in accordance with embodiments of the invention;

Figure 18 is a flowchart of a method of therapy selection using patient response information in accordance with embodiments of the present invention;

Figure 19 is a flowchart illustrating a method of template selection and generation for therapy selection using patient response information in accordance with embodiments of the invention;

Figure 20 is a flow chart illustrating a method of determining patient therapy response information in accordance with embodiments of the invention;

Figure 21 is a block diagram illustrating determination of patient therapy response information in accordance with embodiments of the invention;

Figures 22A and 22B are flowcharts illustrating a method of providing therapy for characterizable and uncharacterizable arrhythmic episodes in accordance with embodiments of the invention;

Figure 22C is a flowchart illustrating a method of modifying arrhythmia discrimination parameters in accordance with embodiments of the invention;

Figures 23A and 23B are flowcharts illustrating a method of associating a successful therapy with a type of arrhythmia in accordance with embodiments of the invention;

Figure 24 illustrates a method of determining if a cardiac beat is correlated to a template in accordance with embodiments of the invention; and

Figure 25 provides graphs illustrating alignment of rate and shock channel signals of a cardiac beat and an arrhythmia template in accordance with embodiments of the invention.

Figure 26 is a flow chart illustrating a method of initializing templates in accordance with embodiments of the invention; and

Figure 27 is a block diagram of a medical system that may be used to implement arrhythmia therapy selection using patient therapy response information and other patient information in accordance with embodiments of the present invention.

While the invention is amenable to various modifications and alternative forms, specifics thereof have been shown by way of example in the drawings and will be described in detail below. It is to be understood, however, that the intention is not to limit the invention to the particular embodiments described.

## DETAILED DESCRIPTION OF VARIOUS EMBODIMENTS

In the following description of the illustrated embodiments, references are made to the accompanying drawings which form a part hereof, and in which is shown by way of illustration, various embodiments in which the invention may be practiced. It is to be understood that other embodiments may be utilized, and structural and functional changes may be made without departing from the scope of the present invention.

Ventricular tachyarrhythmias are fast heart rhythms that arise within one or more ventricles. Atrial tachyarrhythmias, e.g., atrial flutter or atrial fibrillation, are fast heart rhythms that arise within one or more atria. Cardiac signals representative of ventricular or atrial tachyarrhythmic beats may involve a number of different morphologies. Some types of tachyarrhythmia may be monomorphic. Cardiac signals representing a monomorphic tachyarrhythmia may exhibit a fairly regular rhythm and a similar shape or morphology.

Other types of tachyarrhythmia may comprise multi-morphology or polymorphic ventricular tachyarrhythmia. Each beat of a multi-morphology or polymorphic tachyarrhythmia may be different, making the polymorphic tachyarrhythmia difficult to characterize based on morphology. Ventricular fibrillation is an example of a polymorphic ventricular tachyarrhythmia that presents a disorganized, inconsistent morphology.

Episodes of monomorphic tachyarrhythmia may last only a few beats and may produce minimal symptoms. If the cardiac rate is relatively low, the tachyarrhythmia may be tolerated even if sustained for a number of minutes. Tachyarrhythmia may be treated using a variety of therapies. For example, in some cases, ventricular tachycardia (VT) may be effectively treated by pacing at relatively high energy output when compared to bradycardia pacing. Pacing to mitigate VT may involve one or more pacing bursts and is typically denoted anti-tachycardia pacing (ATP). Other types of VT may require a more aggressive therapy, including high energy cardioversion and/or defibrillation shocks. Still other types of VT may terminate spontaneously without therapy.

The most dangerous form of polymorphic ventricular tachyarrhythmia is ventricular fibrillation, which involves very rapid, small-scale, and uncoordinated contractions. The rapid contractions cause a precipitous drop in blood pressure and low cardiac output. Ventricular fibrillation involving heart rates in excess of about 220 beats per minute rarely terminate spontaneously and may be fatal without rapid therapeutic intervention. Typically therapy for ventricular fibrillation involves a series of high energy defibrillation shocks.

Various embodiments of the invention are directed to an automated process for classifying different types of arrhythmia, e.g., atrial or ventricular tachyarrhythmia. For example, each type of arrhythmia may be associated with a representative beat morphology. A detected arrhythmic episode may be classified as a particular type of arrhythmia by comparing a sample of tachyarrhythmic cardiac beats of the arrhythmic episode with a number of representative beat morphologies associated with the various types of arrhythmia. The arrhythmia may be classified as a particular type of arrhythmia if the sample of the arrhythmic cardiac beats is consistent with the representative beat morphology associated with the particular arrhythmia type.

Various embodiments of the invention are directed to delivering a therapy based on the historical success of the therapy. One or more therapies that were previously successful at treating a particular type of arrhythmia may be associated with the representative beat morphology of the particular type of arrhythmia. Future occurrences of the particular type of arrhythmia may be first treated with a previously successful therapy.

A particular therapy may be selected to treat a particular type of VT. For example, if a therapy is determined to have successfully mitigated or terminated a particular type of VT, information identifying and associating the previously successful therapy with the particular type of VT may be stored in the CRM device memory and utilized by the therapy control unit. If a subsequent episode of the particular type of VT is detected, the previously successful therapy may be selected to treat the particular type of VT. Identification of a therapy that will be used for treatment of a particular type of VT may be performed automatically, e.g., by the CRM device, or manually, e.g., by a physician based on electrogram (EGM) or other data.

Devices, and systems implementing arrhythmia classification and therapy selection in accordance with the present invention may incorporate one or more of the features, structures, methods, or combinations thereof described herein below. For example, a medical system may be implemented to include one or more of the features and/or processes described below. It is intended that such a method, device, or system need not include all of the features and functions described herein, but may be implemented to include one or more selected features and functions that provide useful structures and/or functionality.

While some of examples provided herein have generally been described in terms of an implanted or implantable device, the examples can also be implemented in non-implantable devices and systems, or some functionality may be implemented in an implantable device and other functionality may be implemented in a patient-external device where there is communication between the implantable and the patient-external devices.

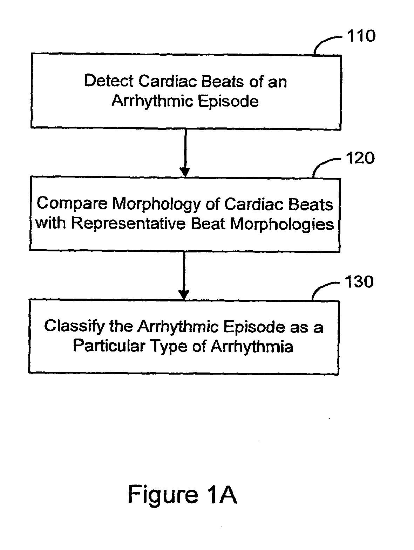

The flowchart of Figure 1A illustrates a method of classifying arrhythmia in accordance with embodiments of the invention. As discussed above, various types of arrhythmia, for example, types of monomorphic atrial tachyarrhythmia or monomorphic ventricular tachyarrhythmia, exhibit characteristic beat morphologies that are relatively consistent from beat to beat for a given patient. The representative beat morphologies of various types of monomorphic tachyarrhythmia experienced by the patient may be identified and used to classify subsequent arrhythmic episodes.

An arrhythmic episode may initially be detected using rate criteria. For example, if a heart rate beyond a predetermined threshold is sensed, the episode may be determined to be a tachyarrhythmic episode. The tachyarrhythmic episode may be further classified using morphology criteria. For example, the tachyarrhythmic episode may exhibit a morphology indicative of a particular type of monomorphic tachyarrhythmia. Cardiac beats of the arrhythmic episode may be detected 110 and compared 120 to representative beat morphologies associated with the various types of arrhythmia of the heart. Atrial beats are used to identify types of atrial arrhythmia, ventricular beats are used to identify types of ventricular arrhythmia. If the morphology of the detected cardiac beats is consistent with (i.e., corresponds with or is sufficiently similar to) a particular type of arrhythmia, then the arrhythmic episode is classified 130 as the particular type of arrhythmia.

In some scenarios, the cardiac beat signals may correspond to two or more representative morphologies. In this scenario, the system may arbitrate between the two or more representative morphologies. For example, arbitration may involve consideration of other factors related to the arrhythmia episode, including, for example, rate of the cardiac beats, drug regimen of the patient, medical information about the patient, neural activity, patient activity, hemodynamic status, cardiac tissue impedance, transthoracic impedance, respiration and/or other factors.

Representative cardiac beat morphologies may be determined based on user input, may be determined automatically, or may be determined using a combination of user input and automatic processing. Representative beat morphologies may be developed using data from a previous device that gathered information from the patient, including cardiac beat signals. Representative beat morphologies may be developed using information gathered from other patients, for example, patients similarly situated and having similar characteristics to the patient. Representative beat morphologies may be developed using externally detected parameters, internally detected parameters, or a combination of externally and internally detected parameters.

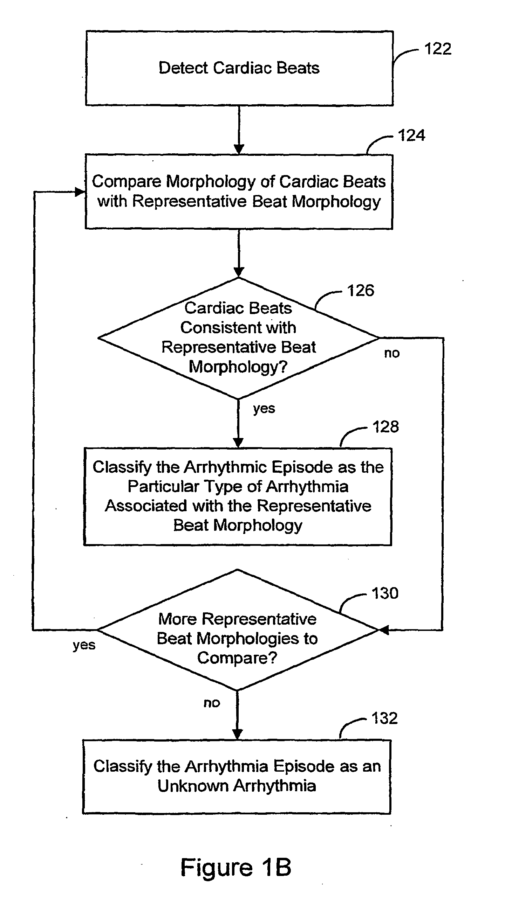

Figure 1B is a flowchart illustrating another method of classifying arrhythmia in accordance with embodiments of the invention. A sample of cardiac beats of an arrhythmic episode is detected 122. The sample of cardiac beats may comprise sequential or non-sequential beats. The morphology of the detected cardiac beats is compared 124 to a first representative beat morphology associated with a type of arrhythmia. If the morphology of the cardiac beats is consistent 126 with the representative beat morphology, then the arrhythmic episode is classified 128 as the particular type of arrhythmia.

If the cardiac beat morphology is not consistent 126 with the representative beat morphology, and there is another representative beat morphology to compare 130, then the next representative beat morphology is compared 124 to the detected cardiac beats.

If the morphology of the detected cardiac beats does not match any of the representative beat morphologies 130, then the arrhythmic episode comprises an arrhythmia of unknown type. The arrhythmic episode is classified 132 as an unknown or unclassified type of arrhythmia. In one example, the unclassified arrhythmic episode may involve a monomorphic arrhythmia that is characterizable by acquiring a representative beat morphology for the monomorphic arrhythmia. In another example, the unclassified arrhythmic episode may involve a polymorphic arrhythmia, such as ventricular fibrillation, for which characterization using a representative beat morphology may not be achievable.

Some current medical devices discriminate between supraventricular tachyarrhythmia (SVT) and ventricular tachyarrhythmia (VT) using cardiac waveform templates. Such devices typically detect a cardiac episode, and compare the episode in question to a normal sinus rhythm (NSR) template using an algorithm that determines a correspondence between episode information and template information. If the episode corresponds with the NSR template, the episode is diagnosed as SVT tachycardia, and therapy is withheld. In contrast, if the episode does not correspond with the NSR template, processes are typically performed to diagnose and verify the episode as ventricular tachycardia, after which therapy may be delivered. In current devices, the particular type of therapy delivered must be decided upon and programmed into the device in advance by the physician. Furthermore, if it is found that a particular type of therapy is not suitable for a patient, the physician must re-program the device during a follow-up visit such that a different type of therapy is delivered for treating the patient's subsequent ventricular tachyarrhythmias.

In varying examples, a morphological template can be generated from a tachyarrhythmia episode and used to identify a later episode. For example, a tachyarrhythmia episode can be detected by an implantable medical device and analyzed to determine whether the episode is a type that necessitates antitachyarrhythmia therapy. In one example, a morphological graph of a tachyarrhythmia episode is presented to a human analyst, such as a physician, and an assessment of the tachyarrhythmia episode is received from the human analyst. The assessment can, for example, include an input that indicates whether the human analyst deems the tachyarrhythmia episode is a ventricular tachyarrhythmia (VT) episode or a supraventricular tachyarrhythmia (SVT) episode. In another example, the tachyarrhythmia episode can be analyzed by a computer to determine whether it is a VT or SVT episode. The computer can be in an implanted medical device, or external to the patient. A morphological template can be generated from the tachyarrhythmia episode and later used to identify a similar episode as it is occurring. For example, if a tachyarrhythmia episode correlates with an SVT template, an SVT can be declared, i.e. the episode can be identified as an SVT and treated accordingly. Templates can be selected or discarded based a variety of factors including conditions in the patient or characteristics of arrhythmias.

In an example, a template comprises one or more points of a sampled data representation of a heart depolarization. In another example, a template is a set of selected features from a sampled data representation of a heart depolarization. In another example a template is generated from multiple SVT beats, for example by taking an average of several beats.

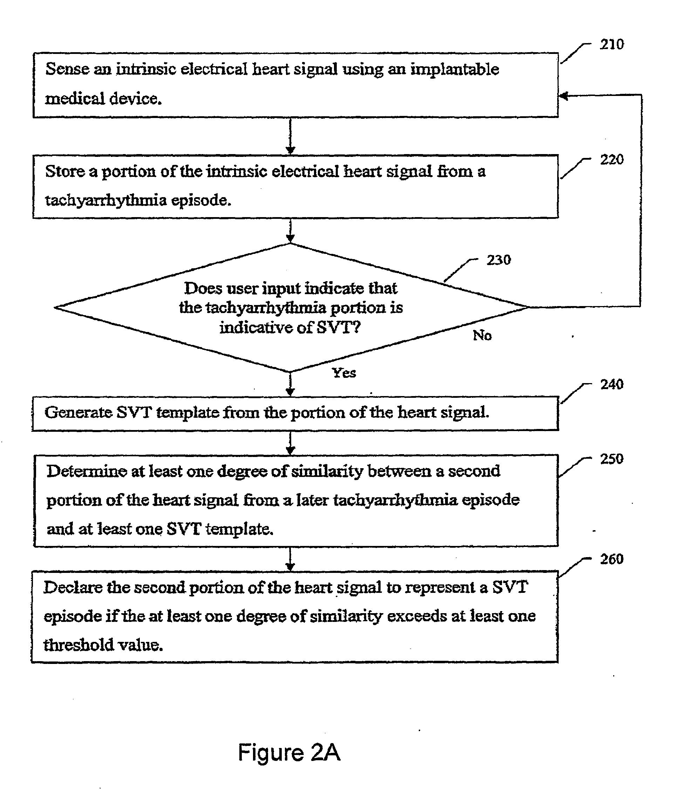

Figure 2A is a flow chart that illustrates a process in which a SVT episode is identified and declared. At 210, an implantable medical device senses an intrinsic heart signal. At 220, the medical device stores a portion of the intrinsic electrical heart signal that was sensed during a tachyarrhythmia episode. At 230, a user indicates whether the portion of the intrinsic electrical heart signal is considered to be indicative of a SVT. In an alternative embodiment, a processor analyzes the portion of the signal to determine whether it is indicative of a SVT. If the portion of the intrinsic electrical heart signal is determined to be indicative of a SVT, a SVT template is generated at 240. In an example, a processor in the implanted medical device generates the template. In another example, the template is generated on an external computer system and downloaded to the implanted medical device. Various techniques can be used to generate the template. In an example, the template consists of one heart beat from the SVT episode. Alternatively, the template includes more than one heart beat. In an example, the template consists of the actual data from the intrinsic electrical heart signal as the template. Alternatively, a processor converts the intrinsic electrical heart signal data into a file that requires less storage space than the original data.

Returning to Figure 2A, at 250, a processor determines at least one degree of similarity between a second portion of the heart signal from a later tachyarrhythmia episode and at least one SVT template. The degree of similarity is a quantity that is computed through an algorithm that uses the sensed intrinsic electrical heart signal (or a derivative thereof) as an input. In varying examples, determining the degree of similarity includes computing a feature correlation coefficient, a wavelet coefficient, or areas or integrals of a heart signal waveform or an approximation thereof. In varying examples, determining the degree of similarity involves computation of data in the time domain, wavelet domain, frequency domain, or other domains.

At 260, the device declares the second portion of the heart signal to represent a SVT episode if the degree of similarity exceeds a threshold valued. In one example, a processor in the medical device declares a SVT episode if a feature correlation coefficient for a heart beat exceeds a threshold. In another example, a processor declares a SVT if multiple feature correlation coefficients for respective multiple beats exceed a threshold value. In one scenario, a SVT is declared if a specified fraction of heart beats in a sequence of beats (e.g. 3 out of 10 beats) exhibit a feature correlation coefficient that exceeds a threshold. In another example, a SVT is declared if an average feature correlation coefficient for a number of beats exceeds a threshold (e.g. the average for 10 beats $\geq 0.94$).

In an example, the processor determines a degree of similarity between the second portion and multiple SVT templates. In one example, the processor begins determining degrees of similarity for the second portion for a sequence of templates and stops determining the degree of similarity if the degree of similarity for one of the templates exceeds a threshold. In other words, the processor compares the heart signal to a sequence of templates until a template match is found or all the templates are used. In another example, the portion of the heart signal is compared to all of the templates regardless of whether a match is found, and an SVT is declared if the degree of similarity for any one of the templates exceeds a threshold.

In addition to supraventricular tachyarrhythmia (SVT) episodes, a heart can exhibit other types of tachyarrhythmia that do not warrant antitachyarrhythmia therapy, including some instances ventricular tachyarrhythmia (VT). Such other non-lethal VT episodes can also be identified and used to generate templates.

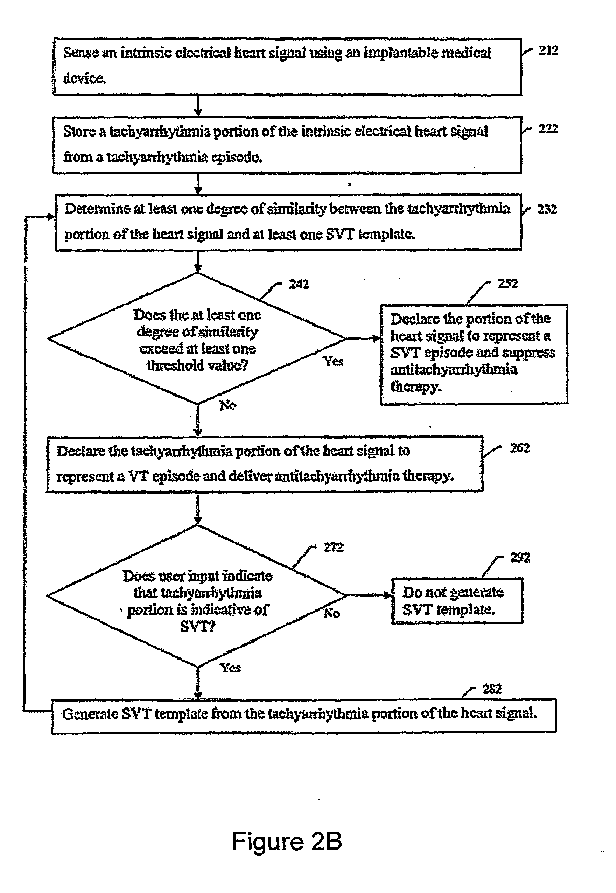

Figure 2B is another flow chart that illustrates a process in which a degree of similarity is determined between a SVT template and a heart signal. At 212, an implantable medical device senses an intrinsic heart signal. At 222, the medical device stores a tachyarrhythmia portion of the intrinsic electrical heart signal that was sensed during a tachyarrhythmia episode. At 232, a processor determines at least one degree of similarity between the tachyarrhythmia portion of the heart signal and at least one SVT template. At 242, the processor determines whether the at least one degree of similarity exceeds at least one threshold value. In an example, the processor determines degrees of similarity for multiple beats and determines whether the degree of similarity exceeds a threshold for at least a predetermined fraction of the beats (e.g. 3 out of 8 beats). If the at least one degree of similarity exceeds at least one threshold value, the portion of the heart signal is declared to represent a SVT episode and antitachyarrhythmia therapy is suppressed, at 252. If the at least one degree of similarity does not exceed at least one threshold value (e.g. fewer than 3 out of 10 beats exhibit a degree of similarity in excess of a threshold), the portion of the heart signal is declared to represent a VT episode and antitachyarrhythmia therapy is delivered, at 262. The portion of the heart signal is stored and later presented to a user. If, at 272, an input from the user indicates that the portion is indicative of SVT, an SVT template is generated at 282. If another tachyarrhythmia event occurs, the process returns to box 232 and determines at least one degree of similarity between the tachyarrhythmia portion of the heart signal from the next tachyarrhythmia event and at least one SVT template, which can be the template generated at 282. If the user input does not indicate that the portion is indicative of SVT, a template is not generated, at 292.

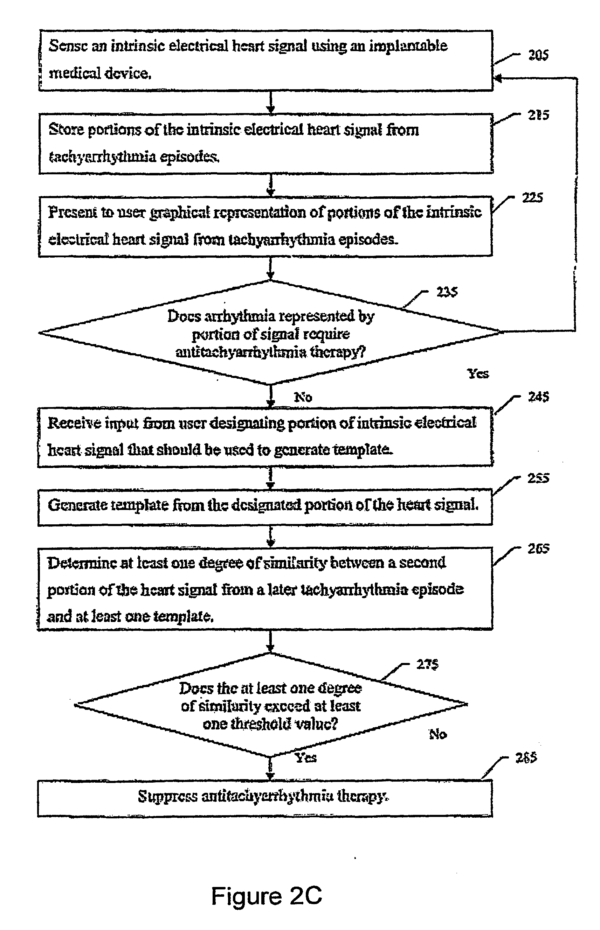

Figure 2C is a flow chart that illustrates a process in which a template is generated for an arrhythmia morphology that does not require antitachyarrhythmia therapy. At 205, an implantable medical device senses an intrinsic heart signal. At 215, the medical device stores one or more portions of the intrinsic electrical heart signal that were sensed during a tachyarrhythmia episode. At 225, a graphical representation of one or more stored portions of the intrinsic electrical heart signal is presented to a user. At 235, the user provides input that identifies at least one tachyarrhythmic portion of the intrinsic electrical heart signal that did not require antitachyarrhythmia therapy. The portion of the intrinsic electrical heart signal that did not require antitachyarrhythmia therapy can be, but is not necessarily, indicative of SVT. At 245, input is received from a user designating at least one portion of the intrinsic electrical heart signal that should be used to generate a template. At 255, a template is generated from a portion of the heart signal designated by the user. In an embodiment, an external computer system receives the input from the user to generate the template, which is downloaded to the implantable medical device. In another example, the user communicates directly with the implantable medical device, which generates the template. In an example, the user designates multiple portions of the heart signal, and multiple templates are generated.

Referring again to Figure 2C, at 265, a processor in the medical device determines one or more degrees of similarity between a second portion of the intrinsic electrical heart signal from a later tachyarrhythmia episode and the template. At 275, the processor determines whether the one or more degrees of similarity exceed a threshold value. If the one or more degrees of similarity exceed a threshold value, the processor suppresses an antitachyarrhythmia therapy at 285.

In an alternative embodiment, in lieu of presenting a graphical representation to a user and receiving an input, a portion of the heart signal is analyzed automatically by a processor to determine whether antitachyarrhythmia therapy is appropriate.

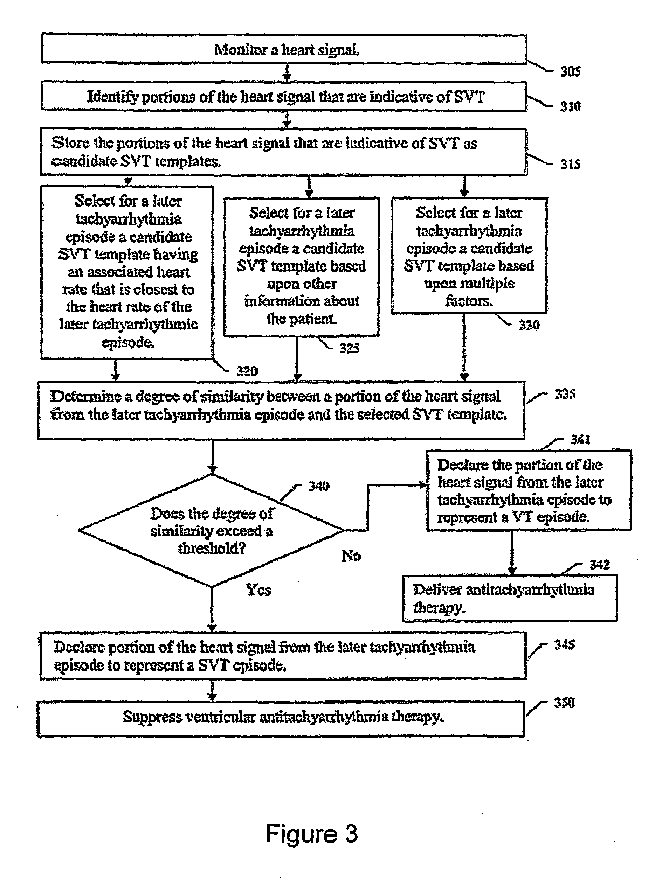

Figure 3 is a flow chart that illustrates a method in which a degree of similarity is determined between a monitored heart signal and a SVT template selected from a group of candidate SVT templates. At 305, a medical device monitors a heart signal. At 310, portions of the heart signal that are indicative of SVT are identified. In varying examples, the portions that are indicative of SVT can be identified by a physician, for example, or identified by an automated computerized analysis. At 315, portions of the heart signal that are indicative of SVT are stored as candidate SVT templates. The candidate template can be stored with a corresponding heart rate at which the template was observed. One or more selection protocols can be used to select a SVT template from a group of candidate SVT templates when analyzing a later tachyarrhythmia episode. For example, at 320, a SVT template is selected from a group of candidate templates based upon the heart rate associated with the candidate templates. The processor selects a template having a heart rate that is closest to the heart rate of the later tachyarrhythmia episode.

Alternatively, at 325, a candidate SVT template is selected based other information about the patient. In an example, the drug treatment regimen of a patient is considered in the selection of a SVT template. The morphology of SVT episodes in a patient can vary depending upon the drug treatment regimen of the patient. In one example, the medical device stores information about drug treatment regimens associated with the tachyarrhythmia episodes used to generate the candidate templates. The current drug regimen is also stored in the device. The processor selects a SVT template based upon a similarity between the drug treatment associated with the selected template and the current drug treatment. In another example, the medical device detects one or more of neural activity, patient activity, sleep state, hemodynamic status, transthoracic impedance, or cardiac impedance. In further examples, the device detects REM (random eye movement) status, sympathetic/parasympathetic tone, intercardiac blood pressure including right ventricular pressure, left atrial pressure, or pulmonary artery pressure, oxygen saturation, heart size and contractability, blood flow, or edema. In varying examples, the medical device considers one or more of these parameters in selecting an SVT template. In another example, the processor identifies one or more characteristics of the patient, and selects a SVT template using statistics from other patients having similar characteristics. In varying examples, the characteristics include (but are not limited to) as height, weight, age, disease history, medication, and patient status (such as neural activity, blood pressure, etc.)

In another alternative, at 330, a candidate SVT template is selected based upon multiple factors. In an example, both the heart rate and the drug treatment regimen are taken into consideration.

At 335, the processor determines a degree of similarity between the heart signal from the later tachyarrhythmia episode and the selected SVT template. The processor compares the degree of similarity to at least one threshold, at 340. If the degree of similarity falls below a threshold, a SVT episode is not declared. In an example, if a SVT episode is not declared, the medical device declares a VT episode at 341 and delivers a ventricular antitachyarrhythmia therapy at 342. Alternatively, other determinations can be made before the medical device administers ventricular antitachyarrhythmia therapy. If the degree of similarity does exceed a threshold, the medical device declares the heart signal from the later tachyarrhythmia episode to represent a SVT episode at 345. The medical device then suppresses ventricular antitachyarrhythmia therapy at 350.

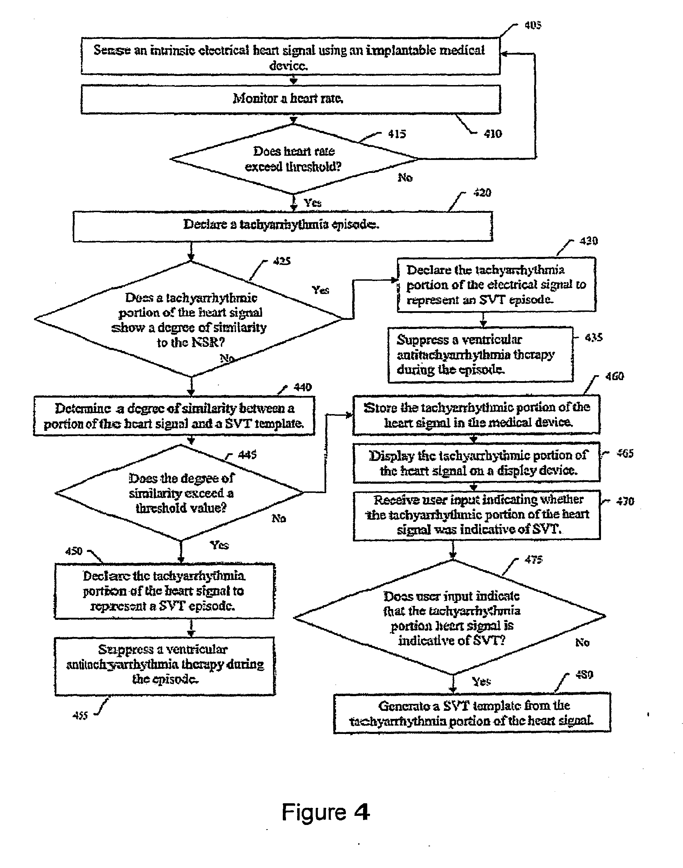

Figure 4 is a flow chart that illustrates a method in which a heart signal from a tachyarrhythmia episode is compared to a normal sinus rhythm and to a SVT template. At 405, a medical device senses an intrinsic electrical heart signal. The medical device monitors the heart rate at 410. At 415, the medical device compares the heart rate to a threshold. If the heart rate does not exceed the threshold, the medical device continues sensing the heart signal but does not perform a tachyarrhythmia discrimination algorithm. If the heart rate does exceed a threshold, the medical device declares a tachyarrhythmia episode at 420. At 425, the medical device compares the tachyarrhythmia portion of the heart signal to the normal sinus rhythm (NSR) template for the patient. In an example, the medical device computes a feature correlation coefficient between the tachyarrhythmia portion of the heart signal and the NSR template. If the tachyarrhythmia portion of the heart signal sufficiently correlates with the NSR template (e.g. the feature correlation coefficient exceeds a threshold), the medical device declares the tachyarrhythmia portion of the heart signal to represent a SVT episode at 430 and suppresses a ventricular antitachyarrhythmia therapy during the episode, at 435.

If the tachyarrhythmia portion of the heart signal is not similar to the NSR template, at 440 the medical device determines a degree of similarity between a portion of the heart signal and a SVT template. At 445, the medical device determines whether the degree of similarity exceeds a threshold. If the degree of similarity exceeds a threshold, the medical device declares the portion of the heart signal to represent a SVT episode at 450. In an example, declaring the portion of the heart signal to represent a SVT episode merely means making a determination to executing a response that is consistent with a SVT, such as suppressing a ventricular antitachyarrhythmia episode at 455.

If, at 445, the degree of similarity does not exceed a threshold, the medical device stores the tachyarrhythmia portion of the heart signal at 460. The stored portion is displayed to a user at 465 and an input is received from the user at 470. At 475, if the user indicates that the heart signal is indicative of SVT, a template is generated from the tachyarrhythmia portion of the heart signal, at 480.

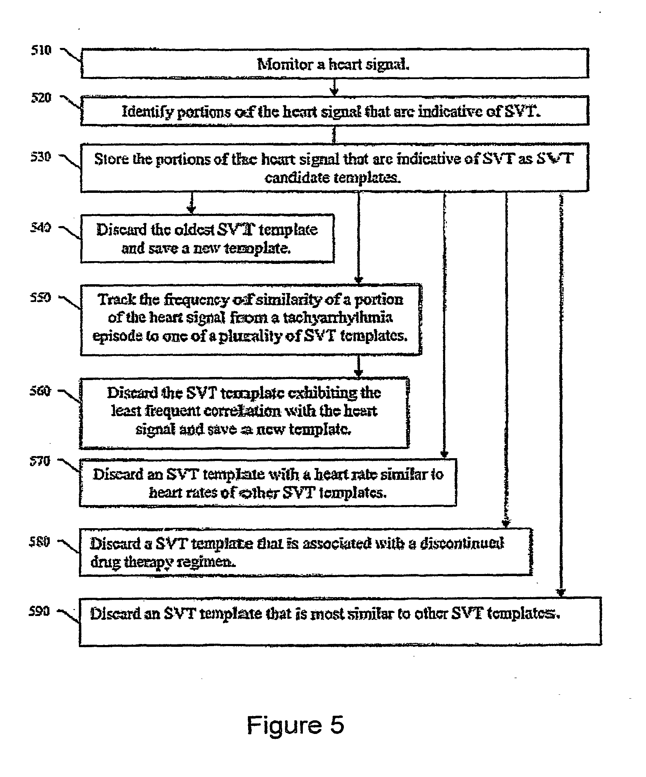

Figure 5 is a flow chart that illustrates examples of methods by which a SVT template is added and an old SVT template is discarded. A medical device monitors a heart signal at 510. Portions of the heart signal that are indicative of SVT are identified at 520 through computer analysis or input from a user. The medical device stores the portions of the heart signal that are indicative of SVT as SVT candidate templates at 530. At 540, the medical device discards the oldest SVT template. Alternatively, the medical device tracks the frequency of similarity of a portion of the heart signal from a tachyarrhythmia episode to one of a plurality of SVT templates at 550 and discards the SVT template exhibiting the least frequent similarity with the heart signal and saves a new template at 560. In another alternative example, the medical device discards a SVT template with a heart rate similar to heart rates of other SVT templates at 570. In the example at 580, the medical device discards a SVT template that is associated with a discontinued drug therapy regimen. In the example at 590, the medical device discards a SVT template that is most correlated to other SVT templates. In varying examples, a decision of which template to discard is made by a computer system, the medical device, or a user who inputs a direction to discard a template.

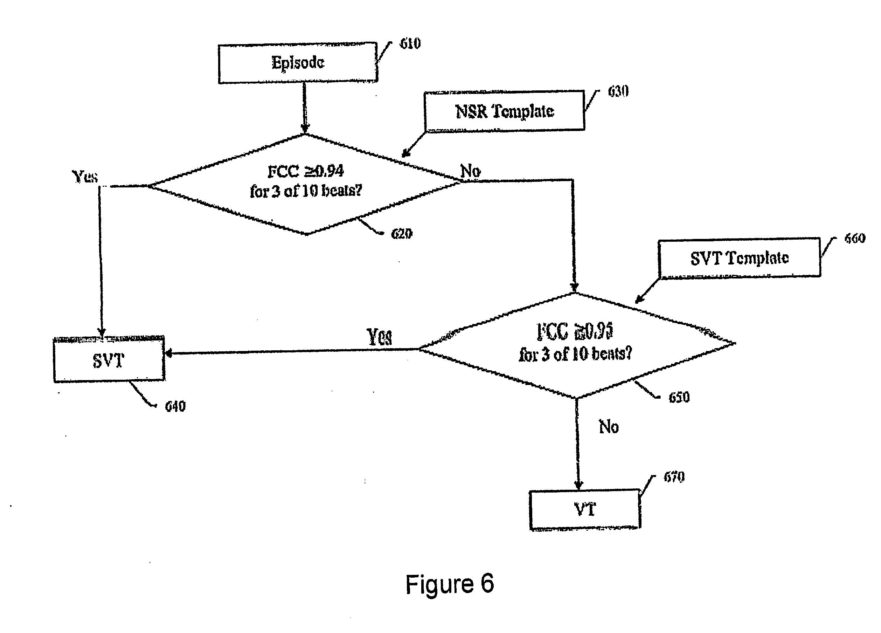

Figure 6 is a flow chart that illustrates an example of a process in which a heart signal from a tachyarrhythmia episode is compared against a normal sinus rhythm (NSR) template 630 and then a supraventricular tachyarrhythmia (SVT) template 660. At 610, a tachyarrhythmia episode is detected. A NSR correlation module 620 computes a NSR feature correlation coefficient (FCC) for 10 heart beats. If the NSR feature correlation coefficient is equal to or greater than 0.94 for 3 out of 10 beats, the correlation module 620 declares a SVT and a SVT protocol 640 is followed. If the fewer than 3 out of 10 beats have a NSR feature correlation coefficient greater than or equal to 0.94, a SVT correlation module 650 computes a SVT feature correlation coefficient for the 10 heart beats. If the SVT feature correlation coefficient is equal to or greater than 0.95 for 3 out of 10 beats, the SVT correlation module 650 declares a SVT. If fewer than 3 out of 10 beats have a SVT feature correlation coefficient equal or greater than 0.95, the episode is treated as a ventricular tachyarrhythmia (VT) and a VT protocol is followed. For example, VT protocol 670 can include administration of anti-tachyarrhythmia therapy.

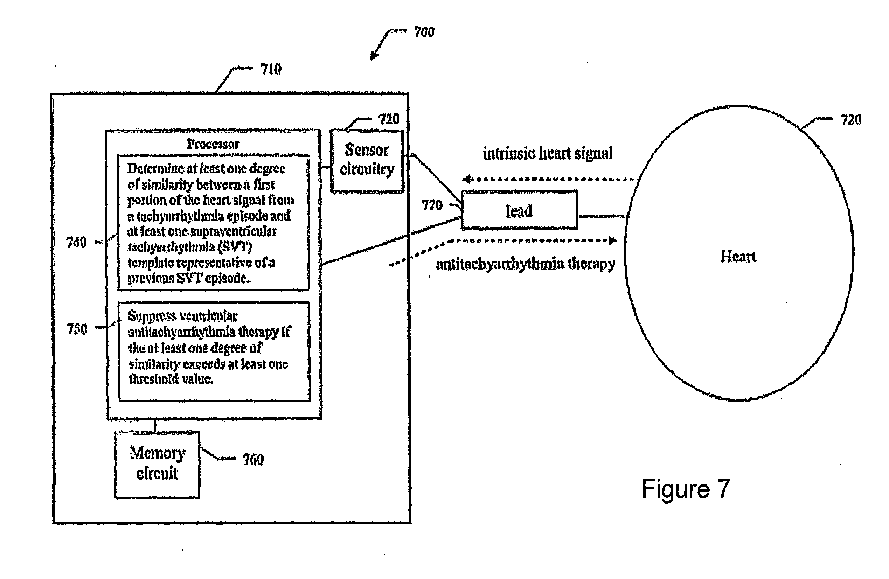

Figure 7 is a block diagram of an exemplary implantable device 700 incorporating a processor 710 and that runs software modules to analyze a signal from a heart 720 through a lead 770. A sensor 720 detects an intrinsic electrical heart signal. The processor 710 receives the signal from the sensor 720 and interprets the signal to determine whether an antitachyarrhythmia therapy should be delivered. A similarity module 740 determines a degree of similarity between portion of the heart signal from a tachyarrhythmia episode and a SVT template representative of a previous SVT episode. A suppress therapy module 750 suppresses a ventricular antitachyarrhythmia therapy if the degree of similarity exceeds a threshold value. SVT templates are stored in a memory circuit 760. A lead 770 delivers ventricular antitachyarrhythmia therapy as directed by the processor.

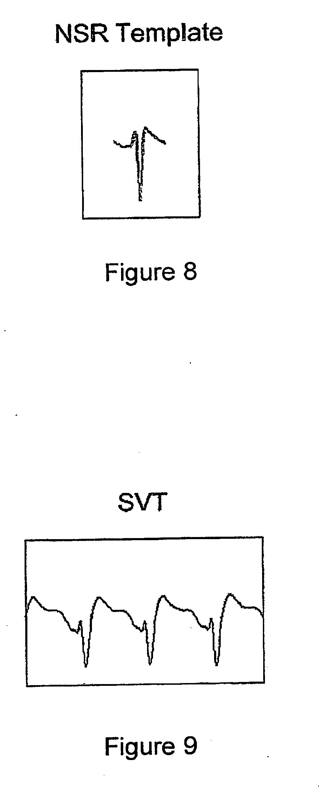

Figure 8 shows an example of a normal sinus rhythm morphology. Figure 9 shows an example of a SVT morphology. In an example, a template includes a morphology for a single beat, as shown in Figure 8. In another example, a template includes multiple beats, as shown if Figure 9. In another example, a template for an atrial tachyarrhythmia can be created. The atrial tachyarrhythmia template can be used to declare a future episode of atrial tachyarrhythmia and suppress antitachyarrhythmia therapy as appropriate.

Varying algorithms can be used to generate a template and determine the degree of similarity between a heart signal and a template. In one example, a set of pre-determined rules are used to locate a number of points on a waveform, and those points are used as the template for comparison against a heart signal. In an example, eight pre-determined points can be located. In one example, the maxima, minima, and inflection points are located. The located points are stored as the template. Features are extracted from a portion of the heart signal by aligning the portion of the heart signal with the template. In an example, a peak on the template is aligned with a peak on the portion of the heart signal from one beat of a tachyarrhythmia episode. The amplitude of the points on the portion of the heart signal is measured at times corresponding to the points in the template. To determine the degree of similarity, a feature correlation coefficient (FCC) is computed using the amplitudes measured from the signal and the amplitude in the template.

In an example, a cardiac rate channel signal and a shock channel signal are sensed. The rate channel signal is measured from a sensing tip to a distal coil on a lead. The shock channel is measured from a distal coil to a proximal coil of the RV lead. A fiducial point is determined from the cardiac rate channel signals. A shock channel signal is aligned using the fiducial point. A template is generated using the aligned shock channel signal. In other examples, a signal is sensed and then aligned using the same channel from which it was sensed.

In another example, a processor computes a wavelet transform or Fourier transform of the signal from which a template is to be generated. A predetermined number of largest wavelet coefficients are saved as a template. Features are extracted from a heart signal by aligning the peak of the signal with the peak of the template and computing the wavelet transform of the aligned tachy beat waveform. A predetermined number of largest wavelet coefficients are saved. The sum of the absolute value of the differences between the saved wavelet coefficients and the wavelet coefficients in the template is determined and divided by the sum of absolute value of the wavelet coefficients in the template. The resulting value is subtracted from 1 and multiplied by 100 to provide a "percent match score", which is compared to a threshold. In an example, the threshold is 70%, and beats having a percent match score greater than or equal to 70% are considered SVT in origin. In an example, if 3 out of 8 beats are SVT, an SVT episode is declared.

In another example, a heart signal waveform is approximated as three consecutive triangles. The areas of each of the triangles is calculated and saved in a template. To extract features from a portion of a heart signal, peaks in the heart signal are aligned with the template, and the heart signal is approximated as three triangles. The area of each of the three triangles is calculated. To determine the degree of similarity to the template, a "similarity score" is determined based on the sum of the differences in the areas of the signal triangles and the template triangle. The similarity score is inversely proportional to this sum. If the similarity score is greater than a threshold, the beat is considered SVT in origin. If a predetermined number of beats exceed a threshold, an SVT episode is declared.

Various embodiments of the invention are directed to delivering an appropriate therapy for a particular of type of arrhythmia experienced by the patient. In accordance with embodiments of the invention, types of arrhythmia are respectively associated with various therapies used to treat the types of arrhythmia. In some embodiments, a therapy is associated with a particular type of arrhythmia if the therapy is determined to be successful at treating the particular type of arrhythmia.

In one implementation, if a particular type of arrhythmia occurs, then a first therapy assigned to treat the particular type of arrhythmia is delivered to the patient. Additional therapies may be applied in the event that the first therapy does not successfully mitigate the arrhythmia. If a therapy other than the first therapy is successful at terminating or mitigating the arrhythmia, the successful therapy may be used as the first therapy to treat subsequent episodes of the particular type of arrhythmia.

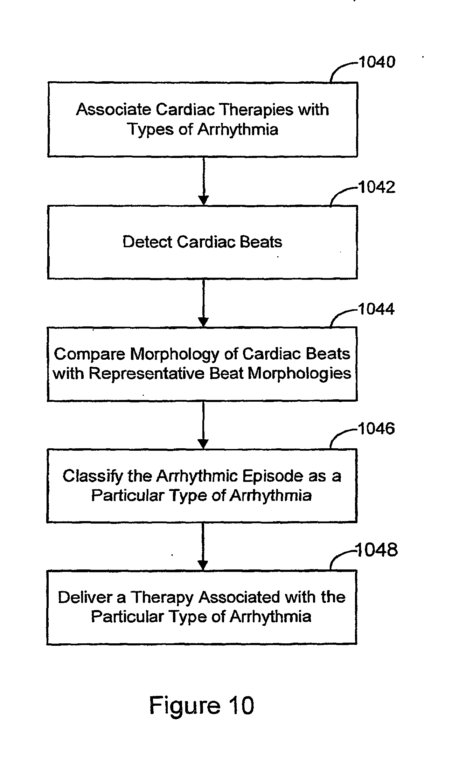

Figure 10 illustrates a method of delivering cardiac therapy to a patient in accordance with embodiments of the invention. Various cardiac therapies are respectively associated 1040 with types of arrhythmia. A representative set of cardiac therapies may involve; for example, antitachycardia pacing (ATP) including burst pacing, (e.g., pacing at 25 Hz or 50 Hz sequences), ramp pacing (e.g., burst pacing with each pace-to-pace interval shortened), scan pacing (e.g., burst pacing with the burst cycle length of each burst shortened between successive bursts, cardioversion shocks (e.g., cardioversion shocks delivered at about 0.5 Joules to about 2 Joules), and titration of defibrillation shocks.

The cardiac beats of an arrhythmic episode are detected 1042. The morphology of the detected cardiac beats is compared 1044 to representative beat morphologies associated with various types of arrhythmia. The arrhythmic episode is classified 1046 as a particular type of arrhythmia based on the comparison. A therapy associated with the particular type of arrhythmia is delivered 1048 to the patient

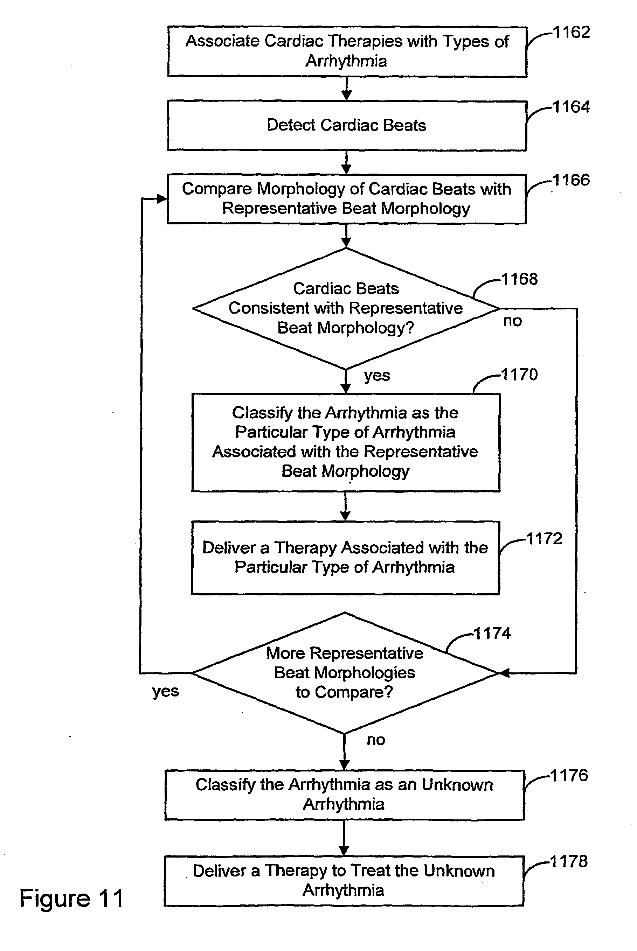

Figure 11 illustrates another method of delivering cardiac therapy to a patient in accordance with embodiments of the invention. Various cardiac therapies are respectively associated 1162 with types of arrhythmia. The cardiac beats of an arrhythmic episode are detected 1164. The morphology of the detected cardiac beats is compared 1166 to a representative beat morphology associated with a type of VT. If the morphologies of the cardiac beats are consistent 1168 with the representative beat morphology, then the arrhythmic episode is classified 1170 as the particular type. A therapy associated with the particular type of arrhythmia is delivered 1172 to the patient.

If the morphologies of the detected cardiac beats do not match any of the representative beat morphologies 1174, then the arrhythmic episode may be classified 1176 as an arrhythmia of unknown type and is an unclassified tachyarrhythmia. In one example, the unclassified arrhythmic episode may involve a monomorphic arrhythmia that is characterizable if a representative beat morphology for the monomorphic arrhythmia were acquired. In another example, the unclassified arrhythmic episode may involve a polymorphic arrhythmia, such as ventricular fibrillation, that is not characterizable using a representative beat morphology. A therapy is delivered 117 8 to treat the unclassified tachyarrhythmia.

According to one scenario, illustrated in Figures 1A and 10, arrhythmia classification may be performed by comparing the morphology of the cardiac beats associated with the arrhythmia episode to representative beat morphologies of various types of arrhythmia. The representative beat morphologies may be characterized by stored morphology templates. The morphology templates comprise one or more features, samples, and/or morphological characteristics of cardiac beat signals representative of particular types of arrhythmia. An arrhythmic episode may be characterized as a particular type of arrhythmia, for example, a monomorphic VT (MVT), by determining that samples of the cardiac beats of the arrhythmic episode are correlated to the samples of a particular template. If the arrhythmic episode can be classified as the particular type of arrhythmia, then an appropriate therapy is delivered. For example, a therapy that was previously successful at terminating or mitigating the particular type of arrhythmia may be delivered to the patient.

In another scenario, illustrated by the flowcharts of Figures 1B and 11, the type of arrhythmia experienced by the patient may not match with any previously stored representative beat morphology and the type of arrhythmic episode is thus unknown. If the tachyarrhythmia is a monomorphic arrhythmia, the type of arrhythmia may be characterizable by acquiring a morphology template representing the beat morphology of the type of arrhythmia. However, if the arrhythmia is polymorphic, a template representing a characteristic beat cannot be acquired.

A therapy may be delivered to treat the unclassified arrhythmia. If the arrhythmia is characterizable, then a first therapy may be delivered. If the arrhythmia is not characterizable, a different therapy may be delivered. Information related to the success of a therapy at treating an unclassified VT may be stored to enhance future treatment of an arrhythmia having similar characteristics.

In one example, a morphology template may be acquired for an unknown but characterizable arrhythmia. A therapy is delivered to treat the arrhythmia. The success of the therapy is determined. A therapy that is successful at treating the arrhythmia may be associated with the type of arrhythmia characterized by the acquired morphology template. If a subsequent episode of arrhythmia comprising cardiac beats that match the morphology template is detected, the episode may be treated using the successful therapy.

In various implementations, success of a therapy may be based on effectiveness of the therapy at mitigating arrhythmia, the length of time the therapy had to by applied to mitigate the arrhythmia, the unnecessary pain caused by the therapy, if any, the power requirements of the therapy, and/or other objective or subjective factors.

Therapies associated with types of arrhythmia and/or used to treat known, unknown, new, characterizable, and/or uncharacterizable arrhythmias may be associated or used based on various factors. For example, therapies may be associated or used based on success of the therapy, the most recent use of the therapy, and/or the frequency of use of the therapy.

Therapies associated with types of arrhythmia and/or used to treat known, unknown, new, characterizable, and/or uncharacterizable arrhythmias may be associated and/or used based on various patient information. For example, such therapies may be associated and/or used based on consideration of various factors, for example, rate of the cardiac beats, drug regimen of the patient, medical information about the patient, neural activity, patient activity, hemodynamic status, cardiac tissue impedance, transthoracic impedance, respiration and/or other factors.

Embodiments of the present system illustrated herein are generally described as being implemented in a cardiac rhythm management (CRM) device which may operate in numerous cardioversion/defibrillation and pacing modes known in the art. Various types of single and multiple chamber CRM devices may be used to implement a number of pacing therapies as are known in the art, in addition to cardioversion/defibrillation therapies. A CRM device may implement various anti-tachyarrhythmia therapies, such as tiered anti-tachyarrhythmia therapies, which may involve performing rate-based and/or morphological tachyarrhythmia discrimination analyses.

It is understood that configurations, features, and combination of features described in the present disclosure can be implemented in a wide range of implantable or external medical devices, and that such embodiments and features are not limited to the particular devices described herein. The systems and methods described herein may be implemented in a wide variety of implantable or external diagnostic and/or therapeutic cardiac devices such as defibrillators, cardioverters, pacemakers, cardiac monitors, and resynchronizers, for example.

Although the present system is described in conjunction with an implantable cardiac defibrillator having a microprocessor-based architecture, it will be understood that the implantable cardiac defibrillator (or other device) may be implemented in any logic-based integrated circuit architecture, if desired.

In one embodiment, the CRM device is an implantable cardioverter/defibrillator configured as a single chamber device that operates to process cardiac waveforms according to a template methodology in accordance with the principles of the present invention. In another embodiment, the CRM device is an implantable cardioverter/defibrillator that is configured as a dual chamber device. In yet another embodiment, the CRM device is an implantable cardioverter/defibrillator configured to sense and/or provide electrical stimulation to multiple heart chambers, for example, both ventricles of the heart, as in a resynchronizer used to treat congestive heart failure (CHF).

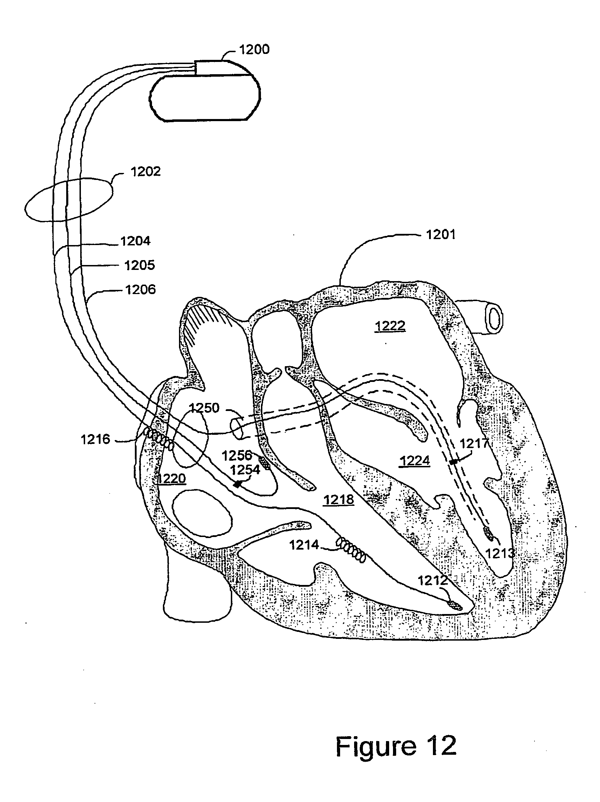

Figure 12 is a partial view of a CRM device that may be used to implement arrhythmia classification and therapy methods in accordance with embodiments of the invention. The CRM device illustrated in Figure 12 includes a pulse generator 1200 electrically and physically coupled to lead system 1202. The lead system 1202 is implanted in a human body with portions of the lead system 1202 inserted into a heart 1201. The lead system 1202 is used to detect electric cardiac signals produced by the heart 1201 and to provide electrical energy to the heart 1201 under predetermined conditions to treat cardiac arrhythmias.

The lead system 1202 includes one or more electrodes used for pacing, sensing, and/or defibrillation. In the particular embodiment shown in Figure 12, the lead system 1202 includes a right ventricular lead system 1204, a right atrial lead system 1205, and a left ventricular lead system 1206. In one embodiment, the right ventricular lead system 1204 is configured as an integrated bipolar pace/shock lead.

The right ventricular lead system 1204 includes an SVC-coil 1216, an RV-coil 1214, and an RV-tip electrode 1212. The RV-coil 1214, which may alternatively be configured as an RV-ring electrode, is spaced apart from the RV-tip electrode 1212, which is a pacing electrode for the right ventricle.

The right atrial lead system 1205 includes a RA-tip electrode 1256 and an RA-ring electrode 1254. The RA-tip 1256 and RA-ring 1254 electrodes may provide pacing pulses to the right atrium of the heart and may also be used to detect cardiac signals from the right atrium. In one configuration, the right atrial lead system 1205 is configured as a J-lead. The lead system 1202 may also include a left atrial lead (not shown) which may be disposed in, on or about the heart to detect electrical signals of the left atrium. In some implementations, for example, the left atrial lead may comprise one or more electrodes attached to the external surface of the heart.

In the configuration of Figure 12, the lead system 1202 is shown positioned within the heart 1201, with the right ventricular lead system 1204 extending through the right atrium 1220 and into the right ventricle 1218. Typical locations for placement of the RV tip electrode are at the RV apex or the RV outflow tract.

In particular, the RV-tip electrode 1212 and RV-coil electrode 1214 are positioned at appropriate locations within the right ventricle 1218. The SVC-coil 1216 is positioned at an appropriate location within the right atrium chamber 1220 of the heart 1201 or a major vein leading to the right atrium chamber 1220 of the heart 1201. The RV-coil 1214 and SVC-coil 1216 depicted in Figure 12 are defibrillation electrodes.

An LV distal electrode 1213, and an LV proximal electrode 1217 may be inserted through the coronary venous system and positioned adjacent to the left ventricle 1224 of the heart 1201. The LV proximal electrode 1217 is spaced apart from the LV distal electrode, 1213 which is a pacing electrode for the left ventricle. The LV distal 1213 and LV proximal 1217 electrodes may also be used for sensing the left ventricle.

The left ventricular lead system 1206 includes endocardial pacing leads that are advanced through the superior vena cava (SVC), the right atrium 1220, the ostium of the coronary sinus, and the coronary sinus 1250 to locate the LV distal 1213 and LV proximal 1217 electrodes at appropriate locations adjacent to the left atrium and ventricle 1222, 1224, respectively.

The left ventricular lead 1206 is guided into the right atrium 1220 of the heart via the superior vena cava. From the right atrium 1220, the left ventricular lead system 1206 is deployed into the coronary sinus ostium, the opening of the coronary sinus 1250. The lead system 1206 is guided through the coronary sinus 1250 to a coronary vein of the left ventricle 1224. This vein is used as an access pathway for leads to reach the surfaces of the left atrium 1222 and the left ventricle 1224 which are not directly accessible from the right side of the heart. Lead placement for the left ventricular lead system 1206 may be achieved via subclavian vein access and a preformed guiding catheter for insertion of the LV electrodes 1213 and 1217 adjacent the left ventricle 1224. In one configuration, the left ventricular lead system 1206 is implemented as a single-pass lead.

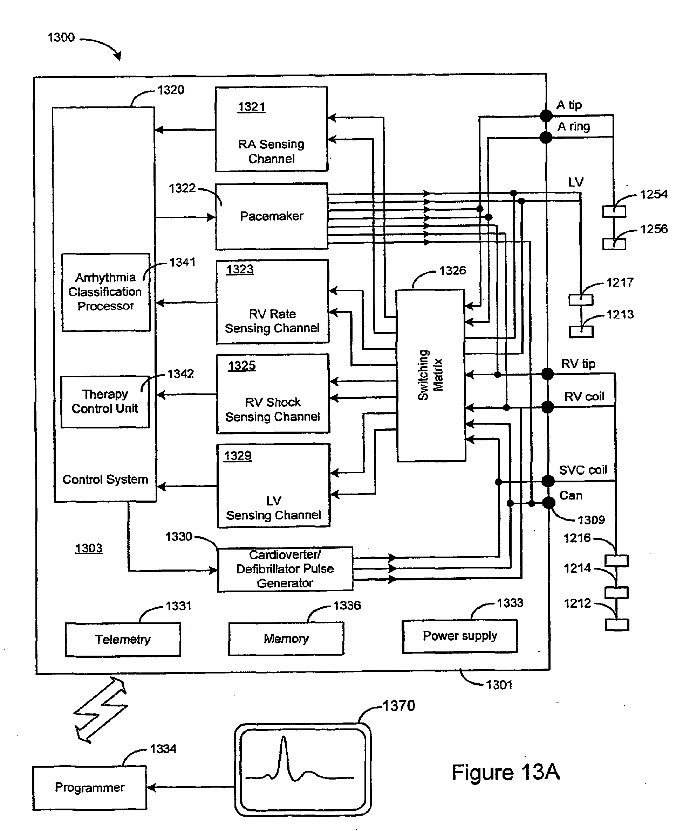

Referring now to Figure 13A, there is shown a block diagram of a cardiac rhythm management (CRM) device 1300 suitable for implementing arrhythmia classification and therapy delivery in accordance with embodiments of the invention. Figure 13A shows a CRM device 1300 divided into functional blocks. It is understood by those skilled in the art that there exist many possible configurations in which these functional blocks can be arranged. The example depicted in Figure 13A is one possible functional arrangement. Various functions of the CRM device 1300 may be accomplished by hardware, software, or a combination of hardware and software.

The CRM device 1300 includes components for sensing cardiac signals from a heart and delivering therapy, e.g., pacing pulses or defibrillation shocks, to the heart. The pulse generator (PG) 1303 of the CRM device 1300 may be encased and hermetically sealed in a housing 1301 suitable for implanting in a human body. Power to the PG 1303 is supplied by an electrochemical battery 1333 that is enclosed within the housing 1301. A connector block with lead terminals (not shown) is additionally attached to housing 1301 to allow for the physical and electrical attachment of the intracardiac lead system conductors to the encased PG 1303.

In one embodiment, the PG 1303 is a programmable microprocessor-based system, including a control system 1320, memory circuit 1336, sensing circuitry 1321, 1323, 1325, 1329, pacemaker 1322, and a cardioverter/defibrillator therapy circuit 1330. Components of the PG 1303 cooperatively perform operations involving arrhythmia classification and therapy delivery according to the approaches of the present invention. The control system 1320 is responsible for arrhythmia detection, classification, and therapy control. The control system 1320 may encompass various functional components, for example, an arrhythmia classification processor 1341 and a therapy control unit 1342.

A memory circuit 1336 may be used to store historical records of sensed cardiac signals, including arrhythmic episodes, and/or information about therapy delivered to the patient. The memory circuit 1336 may be used, for example, to store representative beat morphologies of various types of cardiac signals. Further associations between types of arrhythmia and therapies to treat the arrhythmia types may be stored in the memory 1336.

The historical data stored in memory 1336 may be used for various purposes, including diagnosis of patient diseases or disorders. Analysis of the historical data may be used and/or to adjust the operations of the CRM device 1300. Data stored in the memory 1336 may be transmitted to an external programmer unit 1334 or other computing device, such as an advanced patient management system as needed or desired.

Telemetry circuitry 1331 may be coupled to the PG 1303 to allow the CRM device 1300 to communicate with an external programmer unit 1334 or other remote devices. In one embodiment, the telemetry circuitry 1331 and the programmer unit 1334 use a wire loop antenna and a radio frequency telemetric link to receive and transmit signals and data between the programmer unit 1334 telemetry circuitry 1331. In this manner, programming commands may be transferred to the control system 1320 of the PG 1303 from the programmer unit 1334 during and after implant The remote device may include a display device 1370 capable of displaying various information related to template creation and/or maintenance, and/or cardiac rhythm analysis, such as morphological analysis, rate analysis, wavelet analysis, or other types of rhythm analysis. For example, the display device 1370 may depict a graphical display of one or more detected cardiac waveforms along with the templates used to analyze or classify the detected cardiac waveforms. The display may show various data regarding the number of templates used by the CRM device, including, for example, statistics relating to the frequency particular templates were used to analyze or classify cardiac waveforms. Other uses for the display in connection with the template creation and therapy selection methods of the invention are also possible.

The pacemaker 1322 may be used to deliver a series of electrical stimulations to the heart to regulate heart rhythm. In various embodiments, the pacemaker 1322 may deliver pacing pulses to one or more of the right atrium, left atrium, right ventricle and the left ventricle. The heart may be paced to treat bradycardia, or to synchronize and/or coordinate contractions of the right and left ventricles. The pacemaker 1322 may also provide tachyarrhythmia therapy in the form of anti-tachycardia pacing (ATP) pulses delivered to the heart. The ATP pulses may involve a series of timed paces of programmable width and amplitude that are implemented to interrupt a tachyarrhythmia episode. The ATP therapy may involve, for example, burst pacing at about 25 Hz to about 50 Hz. In various implementations, the pace-to-pace interval may have a variable or constant length.

In the embodiment depicted in Figure 13A, electrodes RA-tip 1256, RA-ring 1254, RV-tip 1212, RV-coil 1214, SVC coil 1216, LV distal electrode 1213, LV proximal electrode 1217, and can 1309 are coupled through a switching matrix 1326 to various sensing circuits 1321, 1323, 1325, 1329. A right atrial sensing channel circuit 1321 serves to detect and amplify electrical signals from the right atrium of the heart. For example, bipolar sensing in the right atrium may be implemented by sensing signals developed between the RA-tip 1256 and RA-ring 1254 electrodes. The switch matrix 1326 is operated to couple the RA-tip 1256 and RA-ring 1254 electrodes to the RA sensing channel circuit 1321 to effect bipolar sensing of right atrial signals. Alternatively, unipolar right atrial sensing may be accomplished by operating the switch matrix 1326 to couple the RA-tip 1256 and can 1309 electrodes to the RA sensing channel circuit 1321.

Cardiac signals sensed through the use of the RV-tip electrode 1212 are right ventricular (RV) near-field signals and are referred to as RV rate channel signals herein. In the system shown in Figure 12, the right ventricular lead is illustrated as an integrated pace/shock lead. In this configuration, bipolar rate channel sensing may be accomplished by operating the switch matrix 1326 to couple the RV-tip 1212 and the RV-coil electrodes 1214 through the RV rate channel sensing circuitry 1323. The rate channel signal may be detected, for example, as a voltage developed between the RV-tip 1212 and the RV-coil 1214 electrodes. The RV rate channel sensing circuitry 1323 serves to sense and amplify the RV rate channel signal.

Unipolar RV sensing may be implemented, for example, by coupling the RV-tip 1212 and can 1309 electrodes to the RV rate channel sensing circuitry 1323. In this configuration, the rate channel signal is detected as a voltage developed between the RV-tip 1212 to can 1309 sensing vector.

The RV lead system may also include an RV-ring electrode (not shown in Figure 13A) used for bipolar pacing and sensing. If an RV-ring electrode is included in the lead system, bipolar sensing may be accomplished by sensing a voltage developed between the RV-tip 1212 and RV-ring (not shown) electrodes.

Far-field signals, such as cardiac signals sensed through use of one of the defibrillation coils or electrodes 1214, 1216 and the can 1309, or using both of the defibrillation coils or electrodes 1214, 1216, are referred to as morphology or shock channel signals herein. The shock channel signal may be detected as a voltage developed between the RV-coil 1214 to the can electrode 1309, the RV-coil 1214 to the SVC-coil 1216, or the RV-coil 1214 to the can electrode 1309 shorted to the SVC-coil 1216. The switch matrix 1326 is operated to couple the desired shock channel sensing vector, e.g., RV-coil to can, to the RV shock channel sensing circuitry 1325. The RV shock channel circuitry 1325 serves to sense and amplify the shock channel signal.

The outputs of the switching matrix 1326 may also be operated to couple selected combinations of the electrodes to LV sensing channel circuitry 1329 for capture detection. For example, the LV proximal electrode 1217 and the LV distal electrode 1213 may be coupled though the switch matrix 1326 to the LV sensing channel circuitry 1329 to sense electrical signals from the left ventricle of the heart. The control system 1320 receives signals from other components of the CRM device 1300 and controls the operation of various CRM functions. The control system 1320 serves as the control for a pacemaker 1322 that delivers pacing pulses to one or more heart chambers. For example, pacing signals may be delivered to selected electrodes according to a preestablished pacing regimen under appropriate conditions.

Unipolar pacing of the right atrium may be accomplished, for example, by delivering pacing pulses between the RA-tip 1256 and can 1309 electrodes. Bipolar pacing of the right atrium may be accomplished by delivering pacing pulses between the RA-tip 1256 and RA-ring 1254 electrodes.

Right ventricular pacing may similarly be implemented using unipolar or bipolar configurations. Unipolar RV pacing involves, for example, pacing pulses delivered between the RV-tip 1212 to can 1309 electrodes. Bipolar pacing involves, for example, delivery of pacing pulses between the RV-tip 1212 to RV-coil 1214 electrodes. If an RV-ring electrode is present, bipolar pacing may be accomplished by delivering the pacing pulses to the RV-tip 1212 and RV-ring (not shown) electrodes.

Left ventricular pacing may be implemented using unipolar or bipolar configurations. Unipolar LV pacing may include, for example, pacing pulses delivered between the LV distal electrode 1213 and the can 1309. Alternatively, bipolar LV pacing may be accomplished by delivering the pacing pulses using the LV distal electrode 1213 and the LV proximal electrode 1217.

The CRM device 1300 includes an arrhythmia classification processor 1341 configured to classify a variety of arrhythmias, including types of monomorphic tachyarrhythmia. The arrhythmia classification processor 1341 may detect and/or classify arrhythmia based on morphological analysis of cardiac signals. The morphological analysis may be performed, for example, by comparing detected atrial or ventricular cardiac beats to stored templates characterizing arrhythmic beats and/or normally conducted beats.

In one implementation, discrimination between supraventricular tachyarrhythmia (SVT) and ventricular tachyarrhythmia (VT) may be accomplished by comparing cardiac beats to a template characterizing the patient's normal supraventricular tachyarrhythmia (SVT). If the cardiac beats are consistent with the SVT template, then the tachyarrhythmia is determined to be supraventricular in origin. In another example, the arrhythmia classification processor 1341 may classify an arrhythmia by comparing the morphology of cardiac beats to templates characterizing ventricular tachycardia, atrial fibrillation and/or atrial flutter. Other arrhythmia detection and/or classification methodologies, e.g., rate based and pattern based arrhythmia detection, are known in the art and may also be implemented by the arrhythmia classification processor 1341.

If an arrhythmia is classified by the arrhythmia classification processor 1341, the control system 1320 may communicate with the cardioverter/defibrillator pulse generator 1330 or the pacemaker 1322 to initiate an appropriate therapy, such as ATP, cardioversion and/or defibrillation shocks, to mitigate or terminate the arrhythmia. The therapy control unit 1342 may associate one or more therapies with types of arrhythmias. For example, if a therapy proves to be successful at treating a particular type of arrhythmia, the therapy control unit associates the therapy with the type of arrhythmia. If the particular type of arrhythmia is subsequently detected, the therapy control unit selects the previously successful therapy to be delivered to the patient.

The therapies delivered to treat the arrhythmia may be delivered in a selected order. The order of therapy delivery may involve delivering a more successful therapy first, followed by other therapies.

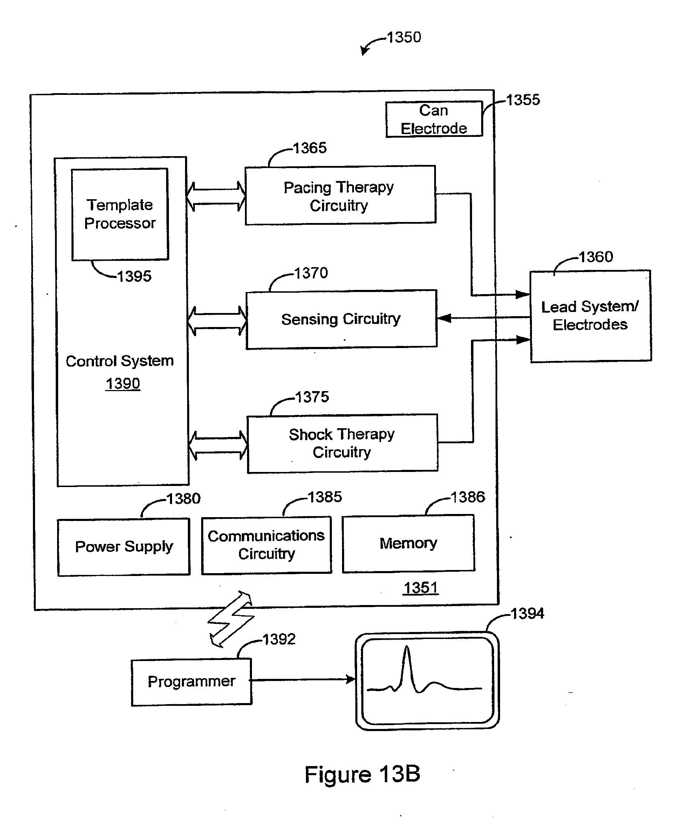

Figure 13B illustrates another configuration of a CRM device 1350 employing a circuitry suitable for implementing therapy selection methodologies of the present invention. Figure 13B shows the CRM device 1350 divided into functional blocks. There exist many possible configurations in which these functional blocks can be arranged. The example depicted in Figure 13B is one possible functional arrangement. The CRM device 1350 includes circuitry for receiving cardiac signals from a heart and delivering electrical energy in the form of pace pulses or cardioversion/defibrillation pulses to the heart.

A cardiac lead system 1360 may be implanted so that cardiac electrodes contact heart tissue as described above in connection with Figure 12. The cardiac electrodes of the lead system 1360 sense cardiac signals associated with electrical activity of the heart. The sensed cardiac signals may be transmitted to a CRM circuitry 1351 through the lead system 1360. The cardiac electrodes and lead system 1360 may be used to deliver electrical stimulation generated by the CRM circuitry 1351 to the heart to mitigate various cardiac arrhythmias. The CRM circuitry 1351, in combination with the cardiac electrodes and lead system 1360, may detect cardiac signals and deliver therapeutic electrical stimulation to any of the left and right ventricles and left and right atria, for example. A can electrode 1355 coupled to a housing of the CRM circuitry 1351 may additionally be used to sense cardiac signals and deliver electrical stimulation to the heart.

In one embodiment, CRM circuitry 1351 is encased in a hermetically sealed housing suitable for implanting in a human body. Power is supplied by an electrochemical battery 1380 disposed within the housing. In one embodiment, the CRM circuitry 1351 is a programmable microprocessor-based system, including a control system 1390, sensing circuit 1370, pacing therapy circuit 1365, shock therapy circuit 13755, and memory 1386. The memory 1386 may be used, for example, to store template information, parameters for various pacing, defibrillation, and sensing modes, and data associated with sensed cardiac signals or other information. The parameters and data stored in the memory 240 may be used on-board for various purposes and/or transmitted via telemetry to an external programmer unit 1392 or other patient-external device, as desired.

The control system 1390 may used to control various subsystems of the CRM device 1350, including the pacing therapy circuit 1365, the shock therapy circuitry 1375, and the sensing circuitry1370. The control system 1390 may also include a template processor 1395 for implementing a template initiation, template generation, template updating, and methodologies for therapy selection according to embodiments of the invention.

Communications circuitry 1385 allows the CRM device 1350 to communicate with an external programmer unit 1392 and/or other patient-external system(s). In one embodiment, the communications circuitry 1385 and the programmer unit 1392 use a wire loop antenna and a radio frequency telemetric link to receive and transmit signals and data between the programmer 1392 and communications circuitry 1385. In this manner, programming commands may be transferred to the CRM circuitry 1351 from the programmer 1392 during and after implant. In addition, stored cardiac data may be transferred to the programmer unit 1392 from the CRM circuitry 1351, for example.

Sensing circuitry 1370 detects cardiac signals sensed at the cardiac electrodes 1360. The sensing circuitry 1370 may include, for example, amplifiers, filters, A/D converters and other signal processing circuitry. Cardiac signals processed by the sensing circuitry 1370 may be communicated the control system 1390 and to the template processor 1395.

The control system 1390 is coupled to the template processor 1395 and uses templates created and maintained by the template processor 1395 to perform various functions, including, for example, arrhythmia analysis and therapy selection. An arrhythmia analysis section of the control system 1390 may compare cardiac signals detected through the sensing circuitry 1370 to the templates created and maintained by the template processor 1395 to detect or predict various cardiac arrhythmias, and to assist selection of appropriate therapies for the patient.

The pacing therapy circuit 1365 is controlled by a pacemaker in the control system 1390 and may be used to deliver pacing stimulation pulses to the heart through one or more of the cardiac electrodes, according to a pre-established pacing regimen under appropriate conditions. Also, the pacing therapy circuit 1365 may deliver ATP therapy in response to VTs that correspond to templates associated with ATP.

The shock therapy circuit 1375 and pacing therapy circuit 1365 are coupled to an arrhythmia analysis section of the control system 1390. The shock therapy circuit 1375 may be used to deliver high-energy electrical stimulation to the heart to terminate or mitigate cardiac arrhythmias such as atrial or ventricular tachycardia or fibrillation detected or predicted by the control system 1390 when patient history suggests that ATP is not effective and/or satisfactory, and/or when a template does not correspond to a cardiac episode.

The CRM 1350 may optionally be coupled to a display device 1394 capable of displaying various information related to template creation and maintenance, and/or cardiac rhythm analysis using morphological templates, as well as other information. For example, the display device 1394 may depict a graphical display of one or more detected cardiac waveforms along with the templates used to analyze or classify the detected cardiac waveforms. The display may show various data regarding the number of templates used by the CRM device 1350, including, for example, statistics relating to the frequency particular templates were used to analyze or classify cardiac waveforms. Other uses for the display in connection with the template creation and adjustment methods of the invention are also possible.

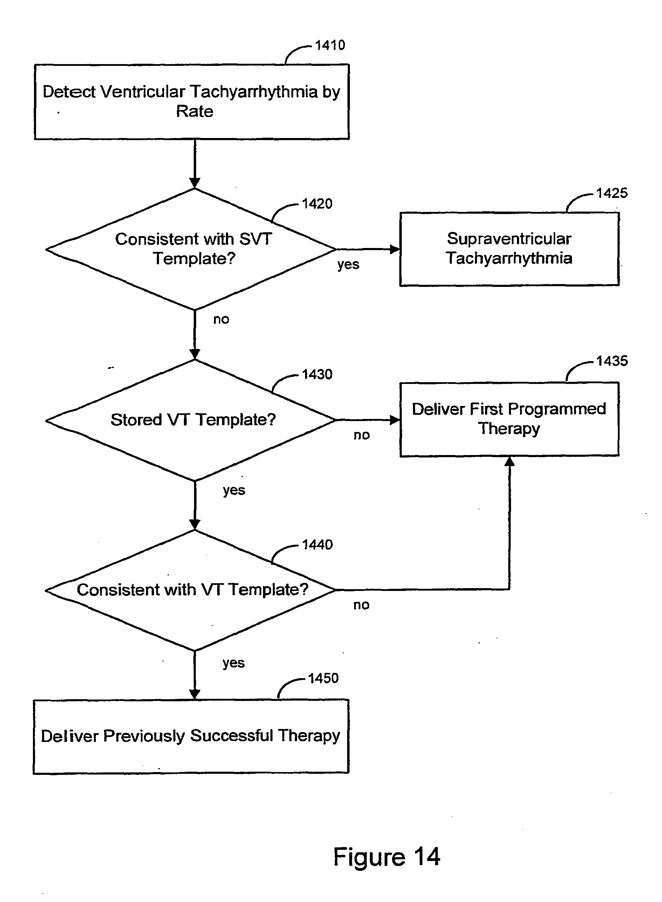

Figure 14 is a flowchart illustrating a method for delivering cardiac therapy to a patient in accordance with embodiments of the invention. An arrhythmia is detected 1410 based on heart rate. For example, tachyarrhythmia may initially be detected based on ventricular rate by evaluating the patient's R-R intervals. If the system detects a ventricular rate above a threshold, then the system flags the episode as a tachyarrhythmic episode. The tachyarrhythmic rate may be categorized as a relatively fast, medium, or slow tachyarrhythmia based on a number or percentage of intervals, e.g., R-R intervals, that fall within specified ranges for relatively fast, medium, or slow tachyarrhythmia.

After the tachyarrhythmia is initially detected 1410 based on rate, a morphological analysis process may be implemented to further classify the type of tachyarrhythmia. In one implementation, the morphology of one or more cardiac beats of the tachyarrhythmic episode is compared to an SVT morphology template characterizing the patient's supraventricular rhythm.

If the one or more cardiac beats are consistent with 1420 the SVT template, then the tachyarrhythmia is classified 1425 as a supraventricular tachyarrhythmia (SVT). In one embodiment, the tachyarrhythmia is classified as an SVT if x out of y beats are consistent with the SVT template. For example, the tachyarrhythmia may be classified as an SVT if at least about 3 out of about 10 beats are consistent with the SVT template. If the tachyarrhythmia is classified as SVT, therapy may not be indicated.

If the morphology of the cardiac beats is not consistent 1420 with the SVT template, then the morphology of the cardiac beats may be compared to previously stored VT templates. If the cardiac beat morphology is consistent with 1440 a VT morphology template, then a therapy that was previously successful at treating the VT is delivered 1450. If the cardiac beat morphology is not consistent with 1440 a VT template, or if there are no previously stored 1430 VT templates, then the system delivers 1435 a programmed therapy.

According to embodiments of present invention, VT templates may be acquired and/or updated and made available for classification of detected arrhythmic episodes. Templates characterizing VT rhythms can only be updated when additional similar VT episodes occur. Templates associated with types of arrhythmia can be acquired or updated automatically or manually. For example, a CRM device may automatically attempt to acquire a VT template upon detection of a rapid ventricular rate that is determined to be ventricular in origin and for which no stored template exists. Alternatively, the physician may identify a VT stored in memory as a past episode and request the implantable system to form a VT template from episode records. In one scenario, the physician may identify a new type of VT and may request that a new VT template be formed. In another scenario, the physician may identify a misclassified VT and request the implantable system to form a VT template for the misclassified VT.

Accurate template formation for a particular type of VT is dependent on the presentation of a consistent beat-to-beat morphology. However, if the cardiac rhythm is disorganized, such as in a polymorphic VT, determination of a representative beat morphology that characterizes the rhythm may not be possible.

A particular therapy may be selected to treat a particular type of VT. For example, if a therapy is determined to have successfully mitigated or terminated a particular type of VT, information identifying and associating the previously successful therapy with the particular type of VT may be stored in the CRM device memory and utilized by the therapy control unit. If a subsequent episode of the particular type of VT is detected, the previously successful therapy may be selected to treat the particular type of VT. Identification of a therapy that will be used for treatment of a particular type of VT may be performed automatically by the CRM device or manually based on electrogram (EGM) data.

Some current medical devices can treat a VT by delivering a high-energy, painful shock or a low-energy, pain-free burst of anti-tachycardia pacing (ATP). Many physicians program these devices to deliver shocks for high-rate VT and reserve ATP for low-rate VT and fast atrial rhythms, such as atrial flutter. However, ATP may be safe and effective for treating high-rate VT, as well.

Furthermore, studies have shown that, for those patients for whom ATP successfully converts their first VT, there is a 99% probability that ATP will successfully convert their subsequent VTs. In contrast, studies have shown that, for those patients for whom ATP does not convert their first VT, the probability of ATP efficacy for subsequent VTs drops to only 38%.

Embodiments of methods and devices in accordance with the present invention may be implemented to adapt to changing patient conditions, medications, and/or cardiac pathology over time. Adaptation over time may possibly decrease the time to effective therapy, and increases the ratio of pain-free to painful therapy, by automatically incorporating information about that patient's prior therapy efficacy when deciding what type of therapy, if any, should be delivered to the current tachyarrhythmia episode.

In various embodiments, cardiac beats may be analyzed by examining the morphology of the electrical cardiac signal waveform. For example, a cardiac beat may be classified as a normal beat, e.g., a normally conducted SVT rhythm, by comparing the cardiac signal waveform to a template characterizing a normal beat. If the cardiac signal waveform is consistent with the template, the cardiac beat may be classified as normal. Similarly, a sensed abnormal or arrhythmic cardiac beat may be classified by comparing the abnormal cardiac signal waveform to one or more templates characterizing an abnormal beat experienced by the patient in the past. The beat may be classified as abnormal if the beat waveform does not match or correspond to a normal beat template and/or if the beat waveform matches or corresponds to an abnormal beat template. Such comparisons may be used to determine that the sensed cardiac beat is abnormal as well as to assess the type of abnormality.

A cardiac waveform template may be created and used to analyze or otherwise process a sensed cardiac signal for a variety of purposes, including, for example, arrhythmia prediction or detection. Cardiac templates may include representative waveforms and/or information derived from waveforms, such as various attributes and/or ranges of attributes of the sensed cardiac signal, including, but not limited to: timing and/or rate information, changes in QRS width, T-wave amplitude, Q-wave amplitude, QT interval, R-R intervals, interval statistics, or other intervals or attributes useful for determining a correspondence between a cardiac waveform and a template. A cardiac waveform template may be formed, for example, by identifying one or more cardiac waveform features representative of a particular cardiac beat morphology. The particular waveform features may include morphological features such as critical points, significant points, curvature, local extrema, inflection points, rise or fall times, slopes, areas above and/or below baselines, and frequency and/or wavelet coefficients, or the like.

In addition to cardiac waveform features, such as waveform morphology, templates in accordance with the present invention may include other information. Patient history to previous therapy, indicating the efficacy of previous therapy attempts for arrhythmias corresponding to a given template, may also be included. Further, other patient information, such as patient activity levels, accelerometer information, hemodynamic status (e.g., blood pressure and blood oxygen level), cardiac tissue impedance, neural activity information, transthoracic impedance, pharmacological agent type and/or level information, respiratory information (e.g., patient disordered breathing information), or other patient information may be associated with a cardiac waveform template in accordance with the present invention. Pharmacological agent type and/or level information may include a change in medications prescribed by a physician, which may alter a patient's heart rhythms, and may require adapting one or more templates to the patient's altered rhythms.

Providing this additional information as a part of the cardiac waveform template enables automated re-programming of implantable medical devices on an episode-by-episode basis, and/or in response to patient-related changes, such as a change in a patient's medication or follow-up from a physician. Information such as medication change information may be provided to an implantable medical device using an advanced patient management system, as will be further described below.

It is understood that the devices and methodologies in accordance with the present invention described with reference to ventricular tachycardia and VT templates are similarly applicable to atrial tachycardia and atrial tachycardia templates, as well as SVT/NSR and SVT/NSR templates. For SVT templates and NSR templates, the therapy associated with the template may be "none" or "withhold therapy" for example. For simplicity of explanation, and not of limitation, the description of devices and methodologies provided herein will generally be made in the context of ventricular tachycardia and VT templates.

Consider, for example, a device configured to treat VT. Once it has been confirmed that a patient has experienced at least one true VT, one or more VT templates may be generated from the prior VT episodes. A VT template may include timing and morphology information, as well as information regarding the type and efficacy of therapy used to treat the prior VT from which the template was generated. The device may use the VT template(s) to automate selection of a therapy for subsequent VTs corresponding to the template(s).

Embodiments of methods and devices in accordance with the present invention may automate this re-programming on an episode-by-episode basis. An episode in question is first compared to one or more templates indicative of a normal supra-ventricular conducted beat, such as an NSR template and/or a template indicative of fast ventricular rhythms originating in the patient's atria. If the episode corresponds to the NSR template and/or the fast atrial-originated template, it is diagnosed as SVT tachycardia (SVT) and therapy is withheld.

However, if the episode does not correspond with the one or more templates indicative of the patient's normal rhythms, the episode is then compared to the VT template(s) created from the patient's prior VT episode(s). If the current episode corresponds with any one of the VT templates, the device then checks the VT template to see if the therapy delivered to the prior VT was satisfactory. If the prior therapy was satisfactory, that same type of therapy is delivered to the current episode, regardless of the rate of the current episode.

In this way, ATP can be delivered to high-rate VTs if ATP was effective at treating similar low-rate VTs in the past. If, however, the therapy delivered to the prior VT was not effective and/or satisfactory, then a different or more aggressive therapy is delivered to the current episode. In this way, the time to effective and/or satisfactory treatment is shortened, as the device does not attempt to use a particular ATP therapy to treat a VT if that ATP therapy was not effective and/or satisfactory at treating similar VTs in the past. The time to effective treatment may be significantly shortened in accordance with an approach that does not attempt to use any type of ATP therapy to treat a VT if a prior ATP therapy was not effective and/or satisfactory at treating similar VTs in the past. In this approach, the device may immediately deliver a cardioversion or defibrillation therapy. In an alternate approach, a particular ATP therapy may be eliminated as an option for treatment if it was not satisfactory, and other ATP therapies may be attempted for subsequent events.

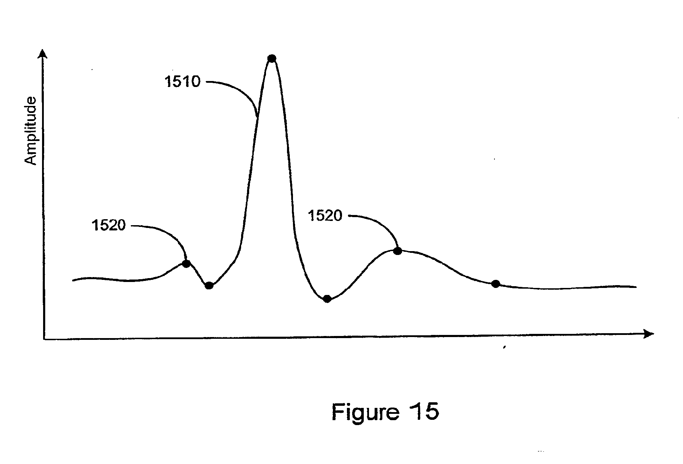

Cardiac beat morphology that may be used to identify SVT and different types of VT may include one of more sample points of the cardiac waveform and/or may include one or more attributes characterizing the cardiac waveform representative of the particular beat morphology, for example. As illustrated in Figure 15, a cardiac waveform 1510 representing a particular beat morphology is sensed and occurrences of one or more cardiac waveform sample points or features 1520 are detected. A waveform feature 1520 may include a particular point of a cardiac signal waveform 1510. The waveform features 1520 may be identified based on various morphological aspects of the cardiac waveform, such as critical points, local extrema, inflection points, rise or fall times, slopes, areas above and/or below a baseline, and frequency and/or wavelet coefficients, or by other aspects, as is known in the art.

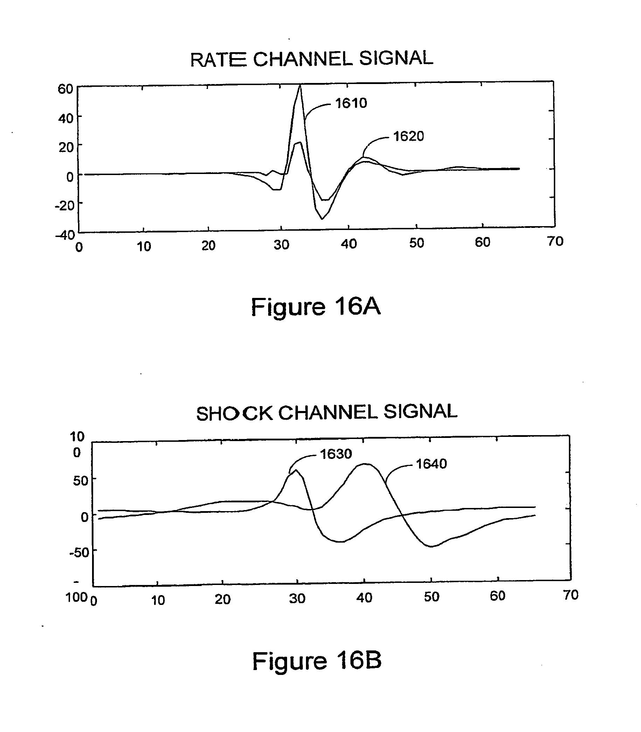

The representative beat morphologies of a number of types of VT may be characterized using morphology templates. In one implementation, template acquisition may be accomplished using a two channel approach. Cardiac beats are sensed on a rate channel and a shock channel. In this example, a feature of the rate channel signal, e.g., the rate channel R-wave peak, may be used to align the shock channels of multiple cardiac beats. Samples or features of the aligned shock channel signals are used to form the template. Figures 16A and 16B illustrate the process of rate and shock channel alignment. Figure 16A illustrates aligned rate channel signals of a normal sinus rhythm (NSR) beat 1610 and a VT beat 1620, respectively. Figure 16B illustrates the aligned shock channel signals of the NSR beat 1630 and the VT beat 1640.

Template formation may involve applying one or more initial criteria to a cardiac beat or a series of cardiac beats before a cardiac beat is used for template formation. For example, the initial criteria may involve meeting various duration, stability, rate, onset, and/or other arrhythmia discrimination criteria prior to using a cardiac beat as a template beat. For example, prior to using a beat as a template beat, the duration of the cardiac rhythm, the rate of the cardiac rhythm, the stability of the cardiac rhythm and/or the onset of the cardiac rhythm may be respectively compared to duration, rate, stability, and onset criteria.

For example, the cardiac rhythm stability may be evaluated by evaluating R—R intervals. A stability analysis algorithm calculates R—R interval differences. The rhythm stability is evaluated by comparing the current average difference to a programmed stability threshold. If the rhythm does not meet stability criteria, then acquisition of a template may not be desirable because unstable rhythms are presumed to be less morphologically consistent than stable rhythms. In this scenario, the unstable rhythm is classified as an unknown rhythm.

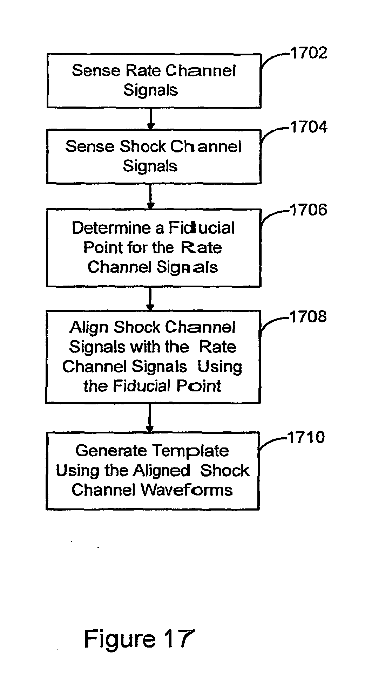

Onset criteria may involve, for example, evaluation of how quickly a particular rhythm occurs. An onset algorithm measures the rate of transition in a rhythm from a first rate to a second rate. If the onset of a cardiac rhythm occurs at a rate that is inconsistent with a threshold rate, then the rhythm may not be suitable for template generation. For example, if the rhythm onset is gradual, the rhythm is likely to be sinus tachycardia and a VT template is not needed. Figure 17 provides a more detailed illustration of the processes associated with forming a template in accordance with embodiments of the invention.

A template may be formed by combining one or more beats, wherein the combination of beats represents one beat of a particular rhythm, such as a monomorphic VT rhythm. Rate channel signals and shock channel signals of one or more cardiac beats representative of the cardiac rhythm being characterized are sensed 1702, 1704. The peaks of the rate channel signals are identified 1706 as fiducial points. The rate channel fiducial points are used to align 1708 the corresponding shock channel signals of each template beat. The aligned shock channel signals may be combined, for example, by averaging the aligned signals sample by sample, or by other methods.

In one implementation, the template may comprise all samples of the average shock channel waveform. In another implementation, a selected set of samples of the average shock channel waveform may be extracted and used to form the template. In yet another implementation, morphological characteristics of the cardiac beats used to form the template may be used as the template. For example, the template may comprise one or more features such as a QRS width, an amplitude, peak timing, and/or other morphological features of the rate channel, shock channel, or combinations thereof

As previously discussed, the representative beat morphology of various types of VT may be acquired manually. Manual identification of representative beat morphology may be accomplished by a physician or other person. For example, the patient's physician may examine stored electrogram (EGM) data acquired by the CRM device or another device to determine morphological features characterizing the representative beat morphology of monomorphic VT episodes. The morphological features may be input to the CRM device for use in identifying subsequent episodes of the type of VT.

Therapies may also be selected and associated with VT types through a manual process. A physician or other person may identify therapies that are likely to be, or have been, most successful at treating various types of VT. Identification of the therapies may be accomplished by examining EGM data corresponding to previous VT episodes and the result of treatment of the VT episodes. A therapy that was previously successful at treating a particular type of VT may be identified by the physician. Knowledge of an effective therapy or therapies may be acquired from a patient's previous device and/or during experience gained during an electrophysiological (EP) study. An association between the particular type of VT and the successful therapy may be input to the CRM device and stored in memory. When the CRM device detects a subsequent episode of the particular type of VT, the previously successful therapy is delivered.

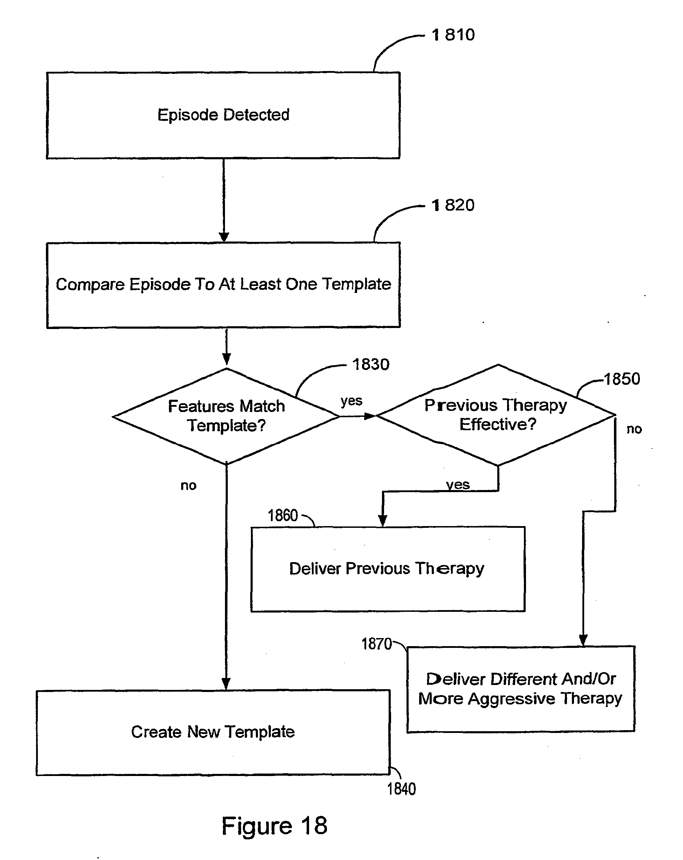

Figure 18 is a flowchart of a method of therapy selection in accordance with embodiments of the present invention. The method begins with an episode 1810 being detected. The episode 1810 may be, for example, a rate-based or other anomaly for which discrimination is desired. Information from the episode corresponds to templates 1820. The information may be a cardiac waveform encompassing a series of beats sensed from one or more electrodes, a portion of a cardiac waveform, a portion of a beat, or attributes from a cardiac waveform, as well as other information such as patient information. As an example, useful for illustrative purposes but not limiting, consider that morphological features correspond 1830 to at least one of the templates 1820, indicating that the episode 1810 is an arrhythmia. The template 1820 that corresponds to the episode 1810 is then used to determine if a previous therapy was satisfactory in treating the arrhythmia. Correspondence to a template may be determined by, for example, correlation, convolution, and/or statistical analysis of cardiac waveform information.

If a previous therapy was satisfactory, for example if the template indicates that ATP was satisfactory in treating the last arrhythmia that corresponded to the template 1820, then the previous therapy is delivered 1860 again. If the previous therapy attempt 1850 was not satisfactory, for example if the template indicates that ATP was not satisfactory in treating the last arrhythmia that corresponded to the template 1820 or if the previous therapy accelerated the cardiac rhythm, then a different and/or more aggressive therapy 1870 is delivered. If no templates 1820 are found to correspond to 1830 the current episode, then a new template may be created 1840, as will be described in more detail below.

Whether or not a particular therapy was satisfactory may be based upon one or more of a variety of factors, including: if the therapy was effective, if the therapy didn't take too long, if the therapy didn't cause unnecessary pain, if the therapy didn't require unnecessary energy, and/or other subjective and/or objective factors.

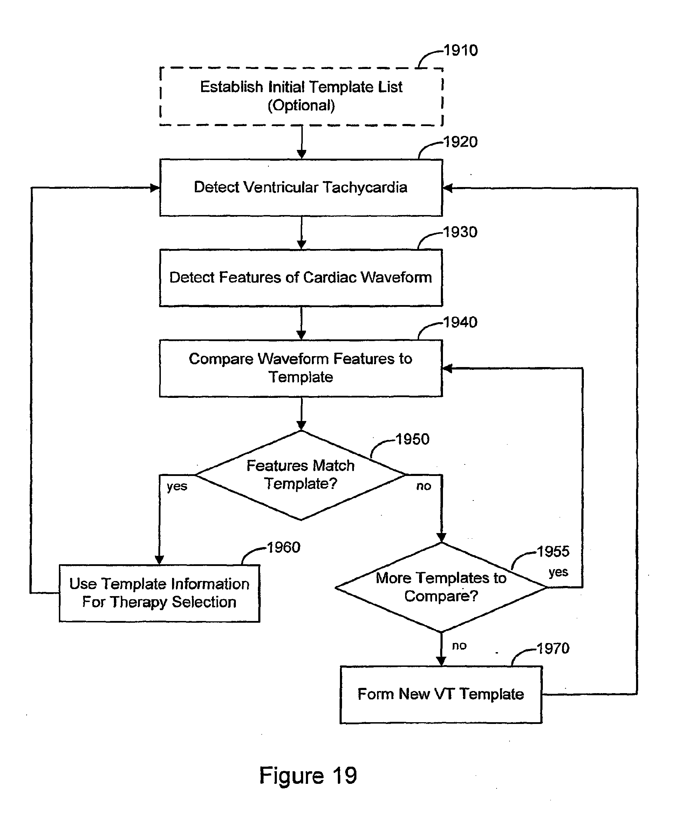

Figure 19 is a flow chart illustrating a method of template selection and generation for therapy selection in accordance with embodiments of the invention. According to this embodiment, an initial template list comprising a list of templates representative of various cardiac beat morphologies and/or characteristics may optionally be established 1910. Alternatively, new templates may be formed without an initial template list using the processes described below.

A ventricular tachycardia is sensed 1920 using a cardiac waveform signal. The waveform features of the cardiac signal are detected 1930 and compared 1940 to each template in the template list, if any. In one example, if a predetermined number of the detected features of the cardiac waveform fall within the target ranges of the template, e.g., six of seven cardiac waveform features fall within target ranges of the template, the cardiac waveform may be classified as matching or corresponding 1950 to the template. The template may then be used for therapy selection 1960, based on patient therapy history information associated with the template, as described above.

If the detected waveform features do not correspond 1950 to a template, the waveform features are compared to the next template until all templates have been compared 1955 to the cardiac waveform features. If none of the templates correspond to the cardiac waveform features, a new template may be created 1970 by, for example, extracting features of the cardiac waveform for correspondence purposes with future sensed episodes, and then associating therapy effectiveness and/or satisfaction information as therapy is delivered to the current episode. Other information may also be associated with the template, such as patient medication levels, patient activity information, and other sensor information measured at or near the time the arrhythmia occurred that may provide improved discrimination for therapy delivery selection.

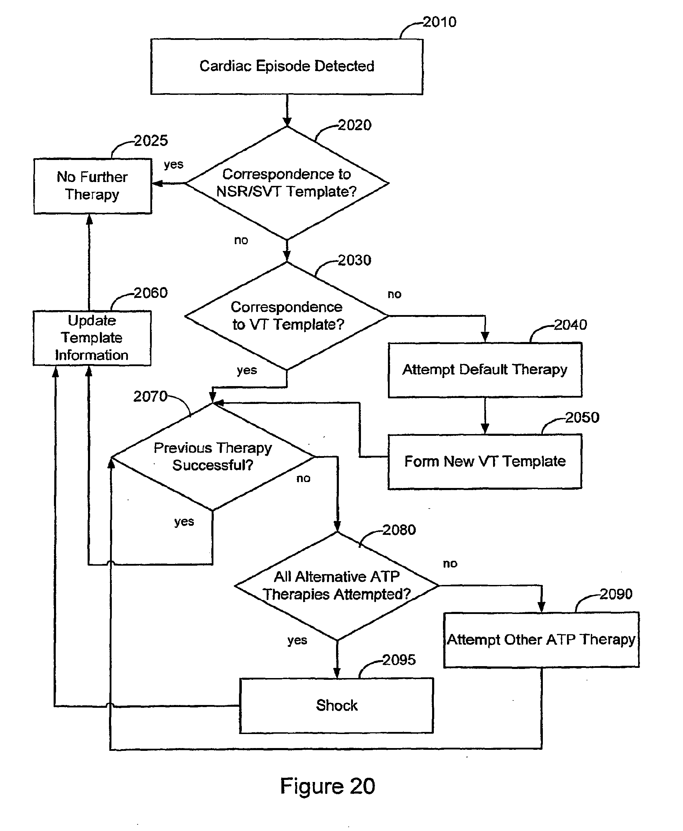

Figure 20 is a flow chart illustrating a method of determining patient therapy response information, such as therapy effectiveness and/or whether or not the therapy was satisfactory, in accordance with embodiments of the invention. The method 600 begins with an episode being detected 2010. The detected episode may be, for example, a rate-based or other anomaly for which discrimination and/or characterization is desired. Information from the detected episode 2010 is compared or matched 2020 to one or more NSR/SVT templates to determine correspondence with the template. One method of determining that a cardiac signal may match, or correspond, to a template utilizes a correlation algorithm or other form of comparison. For example, morphological features of a signal may be measured with respect to amplitudes, inflection points, curvatures, timing of features, or other attributes. Characteristics such as timing onset, stability, variability of intervals between features of successive heartbeats, rates, and other characteristics may be included as part of a template. A template may include acceptable ranges and/or acceptable statistics of measurable features.

As an example, useful for illustrative purposes but not limiting, consider that morphological features are used to match 2020 a set of templates. If the episode 2010 matches or corresponds 2020 to one or more NSR/SVT templates, then the episode is determined to not require therapy 2025.

If the episode 2010 does not match 2020 to one or more NSR/SVT templates, then the morphological features of the episode 2010 are compared 2030 to a set of VT templates. If the morphological features do not match any NSR/SVT templates 620 or any VT templates 2030, then a default therapy 2040 is attempted, and a new VT template may be established 2050. If a maximum number of templates have been reached, the method may erase a template by overwriting it with new information from the latest episode 2010.

In accordance with embodiments of devices that provide communications with a patient-external device, a clinician may be queried to determine if a new template is desired, or if an existing template needs to be updated or erased. This may be accomplished in coordination with an arrhythmia logbook of an implantable medical device, such as a CRM, during patient follow-up. Patient information acquired by such logbook systems may be incorporated or associated with VT (or atrial) templates.

In other embodiments, a template is selected for replacement using a criterion such as: templates older than a predetermined age; the oldest out of all current templates; templates that have not corresponded to an arrhythmia for a pre-determined time period; templates selected for replacement by an advanced patient management (APM) system via manual selection or via algorithmic selection; templates that have corresponded least frequently to an arrhythmia relative to other templates; templates having a pre-determined association (e.g., a drug regimen association); templates having an associated heart-rate or other identified features similar to other templates; or other template selection criteria.

After the new template 2050 is established, patient history information may be acquired to fill in the necessary information of the template for future episodes. If an existing template is matched 2030, or a new template 2050 has been established, a check 2070 determines if the previous therapy was successful and/or satisfactory. The previous therapy may be, for example, a therapy delivered in response to a prior episode, or a therapy previously attempted for the current episode. If the check 2070 determines that the therapy was unsatisfactory, such as by not successfully terminating an arrhythmia, or by accelerating the heart rhythm, then an alternate ATP therapy 2080 may be attempted.

If all alternative ATP therapies 2080 have been attempted, then a cardioversion or defibrillation shock 2095 is delivered to treat the episode 2010, and the template is updated with information 2060 such that the next time a cardiac signal matches that template, ATP will not be attempted, and shock therapy will be chosen. If alternative ATP therapies 2080 have not been tried, then an attempt 2090 is made to determine if a therapy less extreme (e.g., less painful) than cardioversion/defibrillation may correct the episode 610, and another check 2070 is performed. An update 2060 will occur to retain the most recent satisfactory treatment selection determined by the check 2070, such that for any future episode 2010 that corresponds to the template, treatment selection information will be provided for the treatment selection process.

If multiple templates are matched to the episode, then an arbitration process may be initiated to determine which of the multiple templates is to be selected. For example, the most recently used matched template with a successful therapy may be selected as the "best" template. Other criteria may be used to select between multiple matched templates, such as using the template most frequently matched to arrhythmia episodes in the past, the template having the closest rate to the current episode, the template having the highest treatment satisfaction with respect to past arrhythmia episodes, the template having the least aggressive successful treatment, or other useful criterion. The method 600 provides that a CRM incorporating the method may automatically incorporate information about that patient's prior treatment efficacy, and may automatically re-program the CRM on an episode-by-episode basis.

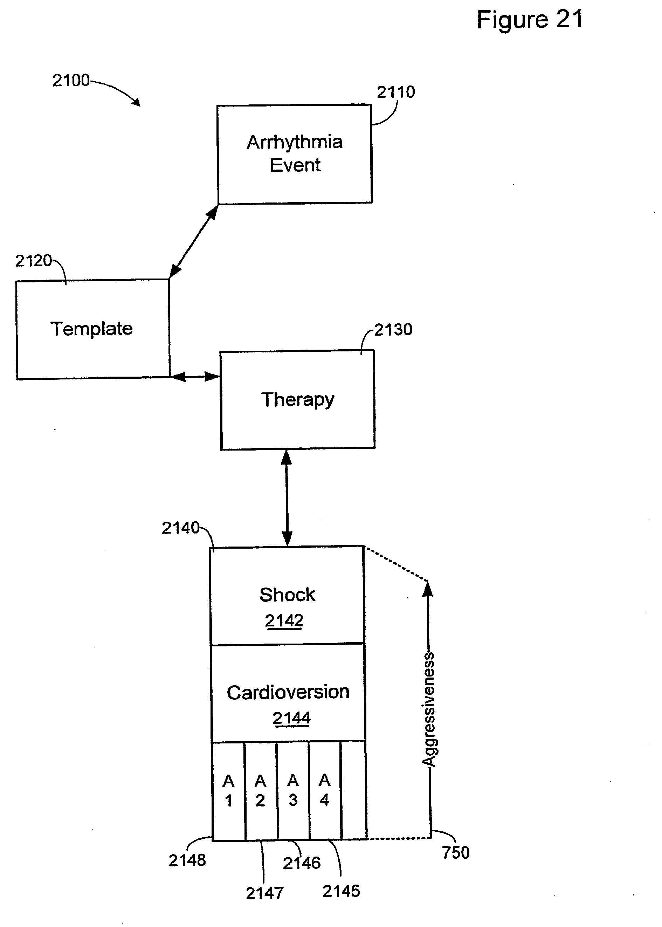

Figure 21 is a block diagram illustrating a methodology for determining patient therapy response information in accordance with embodiments of the invention. An arrhythmia treatment methodology 2100 may be used to associate a therapy with a template in accordance with embodiments of the present invention. The template 2120 may be associated with an arrhythmia event 2110, such as by corresponding to or matching the template 2120, or as having been used to generate the template 212 0. A therapy 2130 is associated with the template 2120.

The therapy 2130 may have been associated with the template 2120 after successfully treating the arrhythmia event 2110. The therapy 2130 may have been delivered to a prior episode, may have been algorithmically selected via an APM system, may have been selected by a physician using a programmer or APM system, or may have been delivered to the current arrhythmia event 2110. For example, an APM system may maintain a database of templates from patients other than the patient experiencing the arrhythmia event 2110. The APM system may find a corresponding template, and determine what was the most satisfactory therapy for treating arrhythmias in other patients. The APM system may then select that most successful therapy as the therapy to associate with the template 2120.

The therapy 2130 associated with the template 2120 may be a single therapy or include a number of therapies, as is shown in Figure 21 as therapies 2140. Therapies 2140 include one or more shock therapies 2142, which may be, for example, a maximum energy shock available from a PIMD, such as a 31 Joule shock (e.g., mono-phasic, bi-phasic, or tri-phasic shocks). Therapies 2140 may also include one or more cardioversion therapies 2144, which may be, for example, a lower energy shock, such as a 14 Joule shock, or other value. Therapies 2140 may also include several ATP therapies, such as ATP1 therapy 2148, ATP2 therapy 2147, ATP3 therapy 2146, and ATP4 therapy 2145. The ATP therapies 2145-2148 may be all different therapies having the same energy level, for example. An aggressiveness level 2150 indicates that ATP therapies 2145-2148 are considered less aggressive than the cardioversion therapy 2144, which is less aggressive than shock therapy 2142.

When a template is originated, an initial arrhythmia may be treated with a lower aggression therapy first, and different therapies, increasing in aggressiveness, may be attempted until a therapy is successful at treating the arrhythmia. The lowest aggressiveness therapy that successfully treats the arrhythmia may then be associated with the template for future reference. This corresponds to a hierarchical approach, where therapies are attempted in an ordered fashion according to an aggressiveness hierarchy.

In other embodiments, a more aggressive therapy may be used when a cardiac waveform is sensed that does not match or correspond to any template, and the waveform may be saved or transmitted for assessment by a clinician to determine if a template should be made, or if another therapy should be associated with that type of cardiac waveform. For example, upon initial determination of a new arrhythmia, a high-energy shock, an initial ATP therapy, or other default therapy, may be used and the cardiac waveform may be designated for review to determine which therapy, if any, should be associated with subsequent corresponding arrhythmias.

In other embodiments, a heuristic approach is used where no hierarchy is established. For example, the last therapy delivered may be repeated, or a therapy may be randomly selected from available therapies until a satisfactory therapy is determined. Satisfaction with a therapy may include parameters other than just effectiveness such as, for example, time to effectiveness, patient pain, patient loss of consciousness, or other satisfaction criteria.

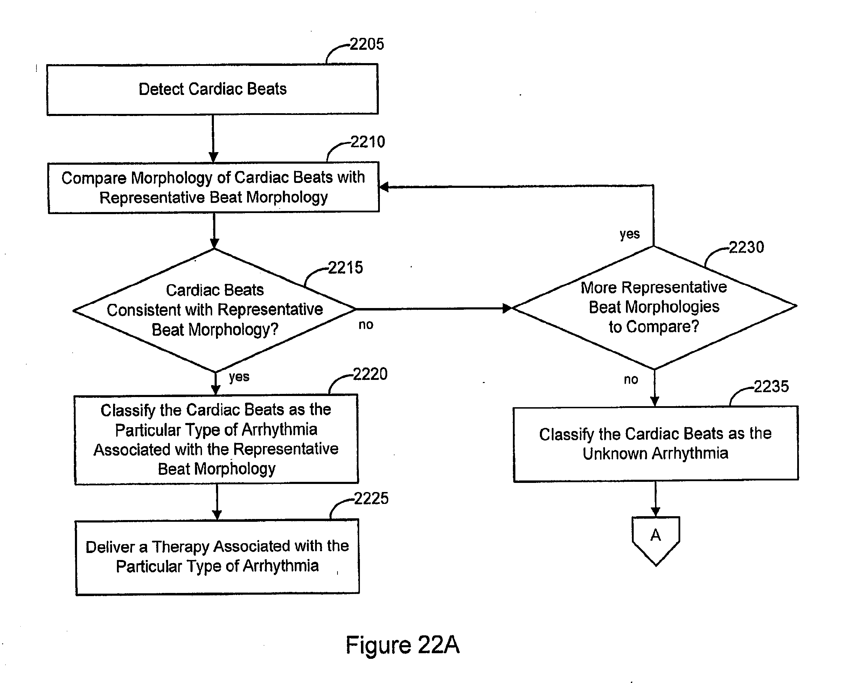

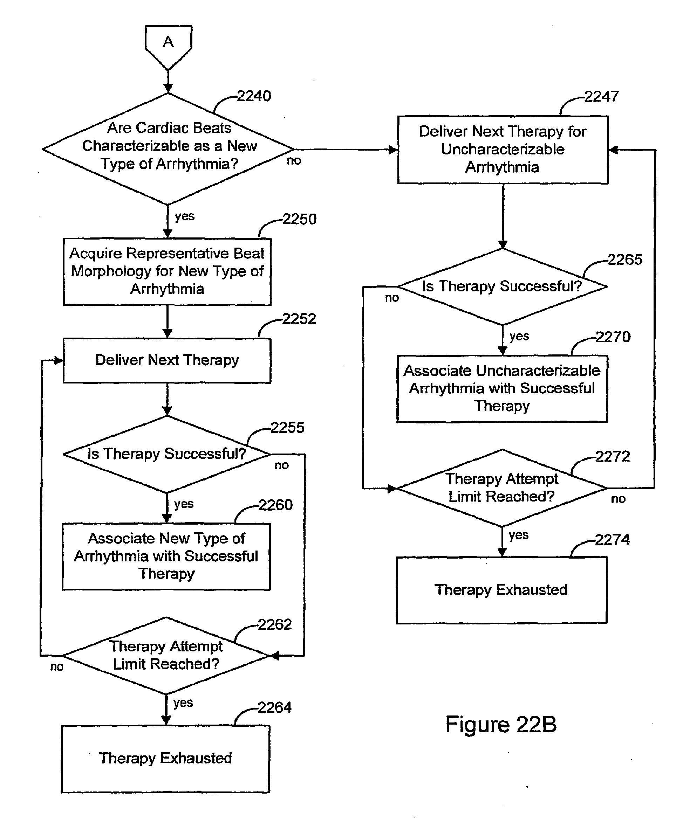

Figures 22A and 22B illustrate a flowchart of a method for delivering therapy to a patient based on recognition of particular types of arrhythmia according to embodiments of the invention. Representative beat morphologies associated with types of arrhythmia may be acquired and stored for use in classification of subsequent arrhythmic episodes. One or more particular therapies may be associated with one or more types of arrhythmia and may be the first therapy used to treat the VT types with which it is associated. In one implementation, additional therapies may be delivered following delivery of the first therapy.

Turning to the flowchart of Figure 22A, cardiac beats of an arrhythmic episode are detected 2205. The morphologies of the cardiac beats are compared 2210 to a representative beat morphology associated with a type of arrhythmia. In one example, the representative beat morphologies are compared to the cardiac beats in a specified order. For example, a representative beat morphology of a more frequently detected type of arrhythmia may be compared to the cardiac beats before other representative beat morphologies are compared. If the morphologies of the cardiac beats are consistent with 2215 a representative beat morphology, then the arrhythmic episode is classified 2220 as the particular type of arrhythmia associated with the representative beat morphology. A therapy associated with the particular type of arrhythmia is delivered 2225.

If the morphologies of the cardiac beats are not consistent 2215 with the particular type of VT, and there are more representative beat morphologies to compare 2230, then the cardiac beat morphologies are compared 2210 to the representative beat morphology of the next type of arrhythmia. If all the stored representative beat morphologies are checked 2230 and the morphologies of the cardiac beats are not consistent with any of the stored representative beat morphologies, then the arrhythmic episode is classified 2235 as an unknown or unclassified type of arrhythmia.

The process of characterizing an unknown type of arrhythmia and identifying a successful therapy continues in Figure 22B. The morphologies of the cardiac beats of the arrhythmic episode may be characterizable 2240 if the arrhythmia comprises a monomorphic tachyarrhythmia having a consistent morphology. However, if the morphologies of the cardiac beats of the arrhythmic episode are uncharacterizable, then the arrhythmic episode is classified as an uncharacterizable arrhythmia, e.g., a polymorphic or multi-morphology VT episode, then a therapy selected for uncharacterizable ventricular tachyarrhythmia may be delivered 2247. If the therapy successfully treats 2265 the uncharacterizable arrhythmia, then the successful therapy may be associated 2270 with uncharacterizable arrhythmia.

If the therapy is not successful 2265 at treating the uncharacterizable arrhythmia, then additional therapies may be tried 2247. The successful therapy, if any, may be associated 2270 with uncharacterizable arrhythmia. The successful therapy may then be selected and used to treat subsequently detected episodes of uncharacterizable arrhythmia. A representative set of cardiac therapies for uncharacterizable arrhythmia such as polymorphic VT or VF may include, for example, burst, ramp, or scan ATP pacing, cardioversion shocks, and defibrillation shocks.

Association of a particular therapy with an uncharacterizable arrhythmia may not always be desirable. For example, ATP may terminate a polymorphic VT, but would be inappropriate for torsade de pointes. Thus, this feature may be disabled on a case by case basis depending on the types of arrhythmias the patient is expected to experience.

The ATP therapy schemes and redetection time should be brief before advancing to therapeutic shock due to minimize duration of any hemodynamic instability associated with the polymorphic rhythm.

If the cardiac beat morphology of the arrhythmia is characterizable 2240, then a representative beat morphology is acquired 2250 and stored for the new type of arrhythmia. A therapy is delivered 2252 to treat the new type of VT. If the first delivered therapy is successful 2255 then the successful therapy is associated 2260 with the new type of arrhythmia, e.g., an arrhythmia for which a representative morphology had not been previously acquired. If the first delivered therapy is not successful 2255, then a next available therapy is delivered 2252. The successful therapy, if any, is associated 2260 with new type of arrhythmia. The successful therapy may then be selected and used to treat subsequently detected episodes of the new type of arrhythmia.

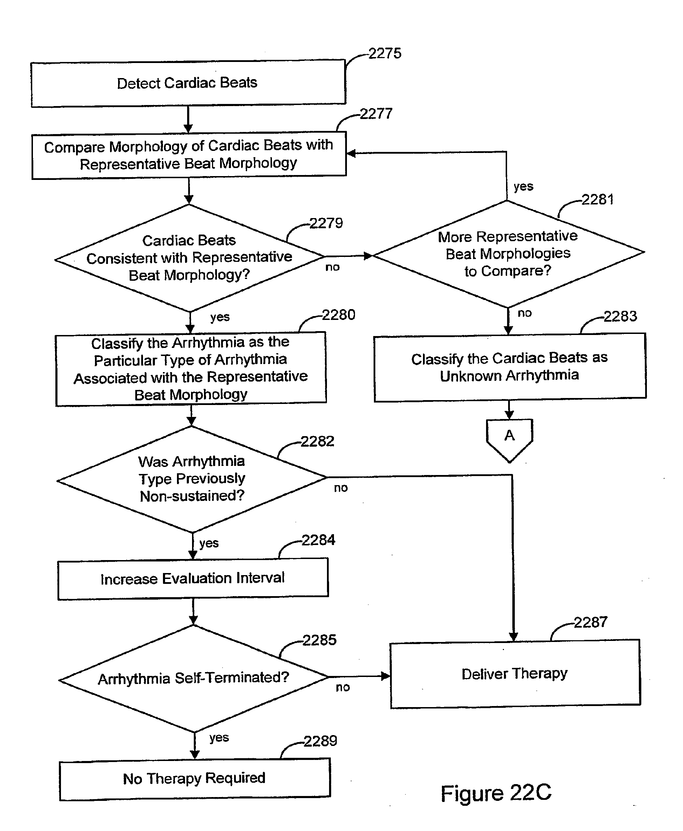

In attempting to acquire templates and/or classify the types of arrhythmia, the CRM device may alter one or more of the arrhythmia discrimination parameters, such as the duration interval or number of cardiac beats used for identifying the rhythm. In one example, the CRM device may learn that certain types of monomorphic arrhythmia or other rhythms may be non-sustained. If a particular type of rhythm is identified as a previously non-sustained arrhythmia type, the device may respond by increasing the duration that an episode is evaluated to allow the arrhythmia episode to self-terminate. For example, a non-sustained VT may last less than about 5 to about 20 seconds, for example. A flowchart illustrating this process is provided in Figure 22C.

Cardiac beats of an arrhythmic episode are detected 2275. The morphologies of the cardiac beats are compared 2277 to a representative beat morphology associated with a particular type of arrhythmia. If the morphologies of the cardiac beats are consistent with 2279 the representative beat morphology, then the arrhythmic episode is classified 2280 as the particular type of arrhythmia.

If the cardiac beats are not consistent with the representative beat morphology 2279 and there are no more representative beats morphologies to compare 2281, the cardiac beats are classified 2283 as an unknown arrhythmia and the process continues as in Figure 22B.

If the particular type of arrhythmia was previously a sustained arrhythmia 2282, then the therapy associated with the particular type of arrhythmia is delivered 2287. However, if the particular type of arrhythmia was previously a non-sustained arrhythmia 2282, then the length of time that the arrhythmia is evaluated may be increased 2284. After the increased evaluation time, the rhythm is rechecked. If the arrhythmia self-terminated 2285, then no therapy is required 2289. If the arrhythmia continues 2285, then the therapy associated with the particular type of arrhythmia is delivered 2287. The type of arrhythmia may be reclassified during or following the increased evaluation interval. In this situation, a therapy associated with the reclassified VT is delivered. The system may update information stored about the arrhythmia, for example whether the arrhythmia was sustained or non-sustained, the duration of the non-sustained rhythms, and/or whether the arrhythmia was reclassified during the increased evaluation period.

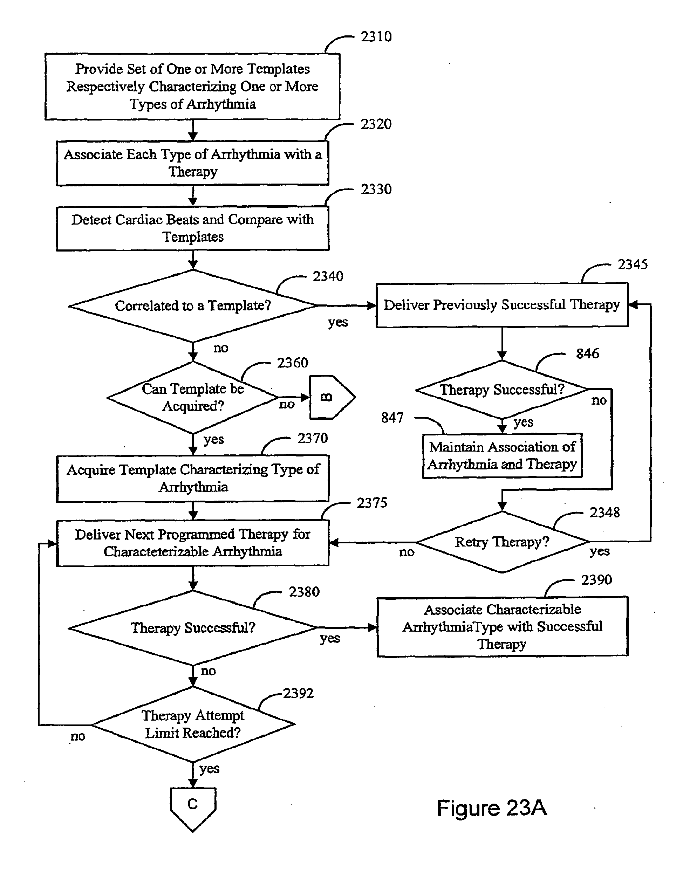

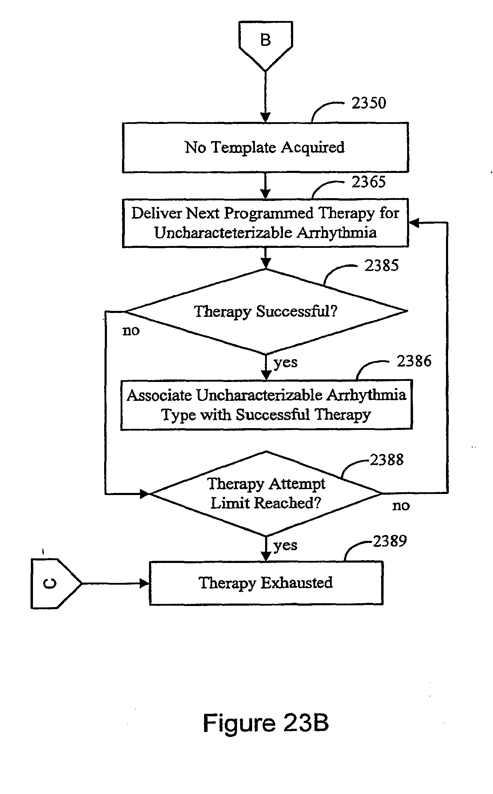

Figures 23A and 23B are flowcharts illustrating a process of using morphology templates to identify types of arrhythmia and to select an appropriate therapy. Morphology templates characterizing one or more types of identified arrhythmia are provided 2310, e.g., stored in memory. In one implementation, each morphology template may be associated 2320 with a particular type of arrhythmia. Each particular type of arrhythmia may be associated with a therapy that is used to treat the particular type of arrhythmia. A therapy may be associated with multiple types of arrhythmia.

Cardiac beats of an arrhythmic episode are detected and compared 2330 to templates characterizing the various types of identified arrhythmias. The order in which the stored templates are compared to the cardiac beats may vary. In one implementation, the order in which the arrhythmia templates are compared to the cardiac beats is determined based on the frequency of occurrence of the types of arrhythmia. For example, the cardiac beats of an arrhythmic episode may be compared to a template characterizing a more frequently occurring type of arrhythmia before the cardiac beats are compared to a template characterizing a less frequently occurring type of arrhythmia. The number of templates compared to the cardiac beat morphology may be limited to a predetermined number of templates.

If the morphology of the detected cardiac beats is correlated 2340 to a stored arrhythmia template, a therapy associated with the type of arrhythmia is delivered 2345. In a preferred implementation, the therapy associated with the type of arrhythmia comprises a previously successful therapy.

If the previously successful therapy is attempted and again successfully terminates 2346 the arrhythmia, the association between the type of arrhythmia and the therapy is maintained 2347. If the previously successful therapy does not terminate 2346 the arrhythmia, then the therapy may be retried 2348 for a predetermined number of times, for example, about 2 times. If the previously successful therapy does not successfully terminate 2346 the arrhythmia after being retried 2348 the predetermined number of times, then the next programmed therapy is delivered 2375.

A number of therapies may be associated with a type of arrhythmia. The therapies delivered to treat the type of arrhythmia may be delivered in a particular order, for example, in the order of a therapy success rating. For example, a therapy determined to be more successful at treating the type of arrhythmia may be delivered first, followed by delivery of the next most successful therapy if the more successful therapy does not mitigate the arrhythmia, and so forth. The therapy success rating for a particular therapy may be determined, for example, based on a percentage of successful treatments using the particular therapy. Thus, if a therapy was previously successful, but becomes less successful at treating the type of arrhythmia, then the previously successful therapy may be replaced by other, more successful therapies. Another example of determining the order of therapies delivered to treat a type of arrhythmia may involve first delivering the one or more therapies that were most recently successful at treating the particular arrhythmia or other arrhythmias, or first delivering the one or more therapies that are most frequently used to treat the particular arrhythmia or other arrhythmias.

If the morphology of the detected cardiac beats is not correlated 2340 to a stored arrhythmia template, an arrhythmia template characterizing the type of arrhythmia may be acquired 2370, if possible 2360. In one implementation, a morphology template may be acquired 2370 by a quick template formation method using about 23 to about 10 cardiac beats.

In some embodiments, illustrated by Figure 23A, a template is acquired 2370, if possible 2360, and a therapy is delivered 2375 to treat the arrhythmia. In other embodiments, a template may be acquired for previously unknown arrhythmias without the delivery of therapy. If the arrhythmia is uncharacterizable 2360, then no template is acquired 2350 (Figure 23B) and therapy to treat the uncharacterizable arrhythmia may be delivered 2365. If the therapy to treat the uncharacterizable arrhythmia is successful 2385, then the therapy may be associated with uncharacterizable arrhythmia 2386 as a successful therapy. Otherwise, the next programmed therapy may be delivered 2365. The system may attempt 2388 a predetermined number of therapies before therapy attempts are exhausted 2389. Information linking a successful therapy with uncharacterizable arrhythmia may be stored in the device memory. The successful therapy may be used to treat subsequent episodes of uncharacterizable arrhythmia as a first selected therapy.

If a template representing the type of arrhythmia was acquired 2370 (Figure 23A) and the therapy delivered 2375 was successful 2380, then information linking the successful therapy to the arrhythmia is stored 2390. Otherwise, the next programmed therapy may be delivered 2375. The system may attempt 2392 a predetermined number of therapies before therapy attempts are exhausted 2389.

Memory available to store templates associated with the arrhythmias may be limited. Thus, templates may be deleted from memory if the arrhythmias they represent have not been detected for a period of time, thus freeing up memory space for more recently detected arrhythmia templates. In one scenario, a newly acquired template may replace a template representing a rhythm that has not been detected for the longest period of time. Further, a manual process for deleting old and/or spurious templates may be used, wherein a physician deletes templates from memory.

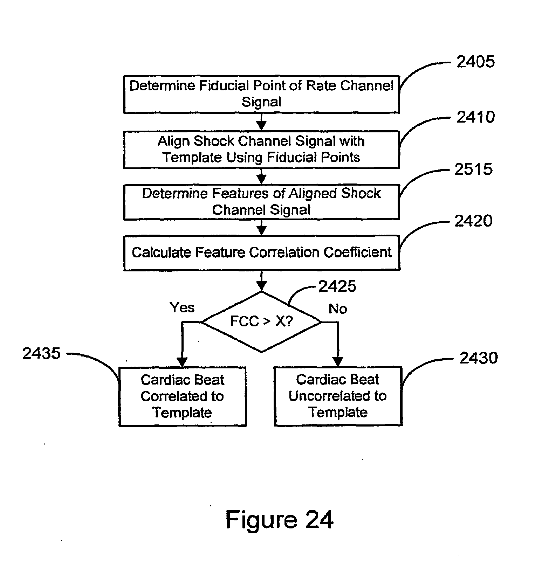

Figure 24 provides a more detailed illustration of various steps associated with determining if a cardiac beat is correlated to a template in accordance with embodiments of the invention. The processes described with reference to Figure 9 may be utilized, for example, in connection with block 2340 of Figure 23. According to one method, template features or samples are identified relative to the fiducial point of the template. The fiducial point, e.g., the R-wave peak, is determined from the rate channel signal of the cardiac beat 2405. The cardiac beat is aligned with the template using the fiducial points of the template and the cardiac beat 2410. A number of features or samples of the cardiac beat are identified at the locations relative to the fiducial point corresponding to previously determined features of samples of the template 2415. The template features or samples and the cardiac beat features or samples may be compared by calculating a feature correlation coefficient (FCC) 2420. In one particular embodiment, Equation 1, provided below, is used to compute the FCC between the template features and the beat features.

$$FCC = \frac{(N\sum_{i=1}^{N}X_i Y_i - (\sum_{i=1}^{N}X_i)(\sum_{i=1}^{N}Y_i))^2}{(N\sum_{i=1}^{N}X_i^2 - (\sum_{i=1}^{N}X_i)^2)(N\sum_{i=1}^{N}Y_i^2 - (\sum_{i=1}^{N}Y_i)^2)} \qquad [1]$$

where, Xi represents template N features and Yi represents beat N features, and N=8 in this illustrative example. The sign of the numerator term is checked before squaring. If the numerator is negative, the beat is uncorrelated, and the remainder of the computation need not be performed.

If the FCC is greater than a predetermined value, as tested at block 2425, for example, about 0.8 to about 0.9, then the cardiac beat is correlated 2435 to the template. If the FCC is less than or equal to the predetermined value, then the cardiac beat is uncorrelated 2430 to the template. The FCC threshold may be configured to be modified by the physician to tailor the sensitivity and specificity of VT recognition to the needs of a particular patient. Increasing the FCC threshold will require a tighter morphological match and will help discriminate between differing VT morphologies. Lowering the FCC threshold will increase the range of related morphologies that will be associated and treated with a particular therapy.

Alternatively, a generalized equation may be used for computation of a correlation coefficient in accordance with a correlation waveform analysis (CWA) technique. An equation for calculation of the correlation coefficient (CC) using this technique may be determined according to Equation 2.

$$CC = \frac{N\sum_{i=1}^{N}X_i Y_i - (\sum_{i=1}^{N}X_i)(\sum_{i=1}^{N}Y_i)}{\sqrt{(N\sum_{i=1}^{N}X_i^2 - (\sum_{i=1}^{N}X_i)^2)(N\sum_{i=1}^{N}Y_i^2 - (\sum_{i=1}^{N}Y_i)^2)}} \qquad [2]$$

where, Xi represents template N samples and Yi represents signal N samples in this illustrative example.

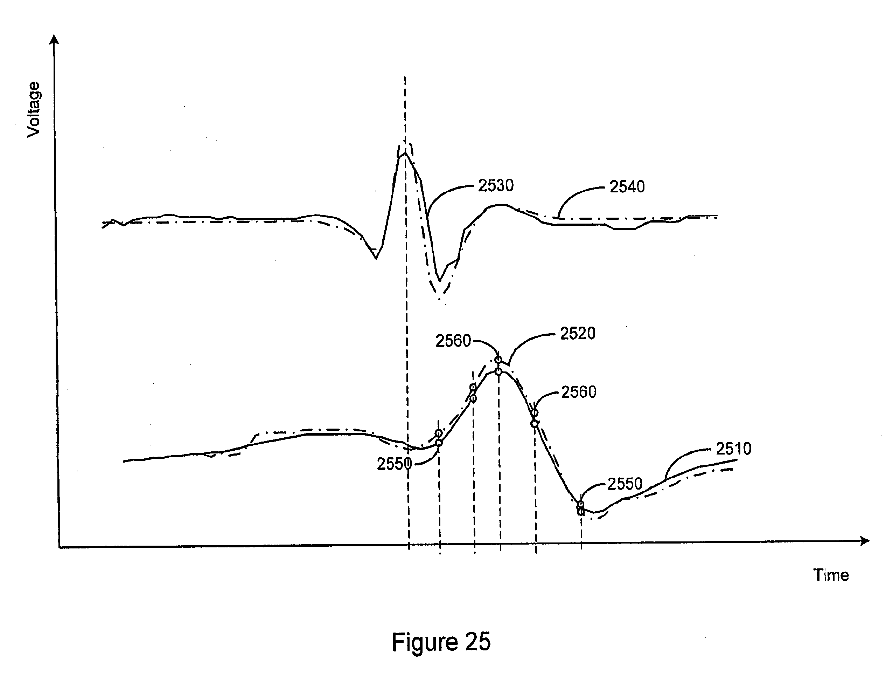

Figure 25 provides graphs illustrating rate and shock channel signals corresponding to a VT template and a cardiac beat. To determine correlation between the cardiac beat and the VT template, the shock channel signals of the template 2510 and the cardiac beat 2520 are aligned based on the R-wave peak of the rate channel signals 2530, 2540. Sample points 2550 of the cardiac beat shock channel signal are compared to template sample points 2560. In one implementation, the feature correlation coefficient between the template and the cardiac beat may be calculated as described above using the selected sample points 2550, 2560. If the feature correlation coefficient is greater than a predetermined value, then the cardiac beat is determined to be correlated to the template. If the feature correlation coefficient is less than or equal to the predetermined value, then the cardiac beat is determined to be uncorrelated to the template.

In some situations, modification of one or more arrhythmia discrimination parameters may facilitate classification of types of cardiac rhythms, such as atrial arrhythmia, ventricular arrhythmia, monomorphic VT, and polymorphic VT rhythms. Retrospective analysis of the classification of cardiac rhythms may reveal that some rhythms were misclassified. Misclassification may occur, for example, if an SVT is misclassified as a VT or if a VT is misclassified as an SVT. Misclassification may also occur, for example, if a characterizable arrhythmia is classified as an uncharacterizable arrhythmia or if an uncharacterizable, polymorphic arrhythmia is classified as a characterizable monomorphic arrhythmia.

In some situations, classification may be enhanced by adjusting one or more arrhythmia discrimination parameters. For example, arrhythmia discrimination parameters may involve the number of cardiac beats used to classify the cardiac rhythm, the beat-to-beat stability of the cardiac beats used to classify the rhythm, the rate of the arrhythmia and/or other parameters used in connection with rhythm classification. Rhythm classification may be enhanced by modifying any or all of the arrhythmia discrimination parameters. The rhythm discrimination parameters that improve the identification of various rhythms represent rhythm characteristics that could also be used to further subclassify arrhythmias, and, as a result, fine-tune therapeutic regimens associated with the arrhythmias.

It is believed that the processes described herein may be utilized in connection with many cardiac beat morphology characterization methodologies.

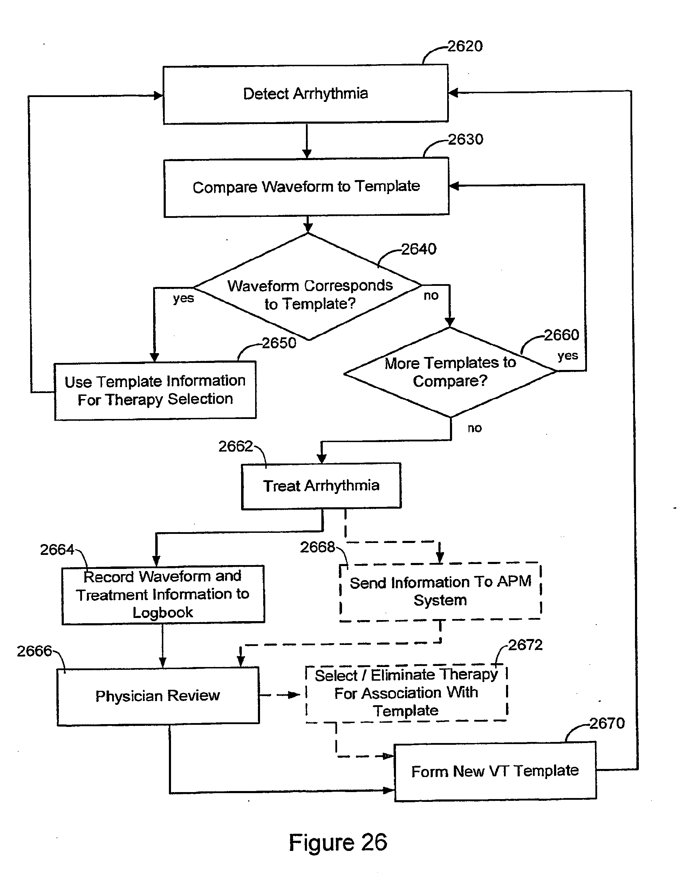

Figure 26 is a flow chart illustrating a method of template selection and generation for therapy selection using patient response information in accordance with embodiments of the invention. According to this embodiment, templates are established by a physician selecting a cardiac waveform for template creation after reviewing a logbook of arrhythmia episodes and treatments. An arrhythmia is detected 2620 using a cardiac waveform signal. The waveform is compared 2630 to each template in the template list, if any. If the cardiac waveform corresponds to a template 2640, the template may then be used for therapy selection 2650, based on patient therapy history information associated with the template, as described above.

If the detected waveform does not correspond to the template 2640, the waveform is compared to the next template until all templates have been compared 2660 to the cardiac waveform. If none of the templates 2640 correspond to the cardiac waveform, the arrhythmia is treated 2662. Treatment 2662 may be performed using one or more of the ATP methods previously described, for example, using a cardioversion and/or defibrillation methodology, or other predetermined default treatment. The cardiac waveform, and any associated information, such as treatment history, and/or other sensor information is recorded 2664, such as by using a memory or logbook feature.

The cardiac waveform and associated information is then reviewed by a clinician, such as a physician 2666. The physician 2666 may select particular recorded waveforms for template generation 2670. The selected waveforms then have templates formed 2670, and a therapy is associated with the template. When reviewing the recording 2664, the physician may select or eliminate from consideration one or more therapies 2672 for association with the template. For example, the physician may determine that a defibrillation was performed on a cardiac waveform that may be treated by ATP. The physician may select ATP therapy as the associated therapy for the template 2670, such that the next arrhythmia associated with a morphology that matches or corresponds to the template 2670 is treated with ATP therapy. The physician may also select an order of multiple therapies to try, for example.

The physician may select an ATP therapy of similar aggression, but different coupling interval. The coupling interval is the point in time during the cardiac cycle at which the pacing pulse should be delivered. A typical coupling interval is 80%, with the pacing pulse delivered at a time predicted to be 80% between the last R wave and the next R wave. ATP with a coupling interval of 70% may deliver the same energy to the patient as ATP with a coupling interval of 80%. However, shorter coupling intervals are considered to be more aggressive, as there is more risk that the pacing pulse could accelerate the rhythm instead of terminating the rhythm.

Other information may also be associated with the template, such as patient medication levels, patient activity information, and other information provided by the physician that may provide improved discrimination for therapy delivery selection. The physician may provide the information during a follow-up office visit, using a programmer, for example. The physician or a clinician may also perform the method of Figure 8 remotely, such as by using an advanced patient management (APM) system 2668 as will be described below. In other embodiments, the APM system may incorporate an artificial intelligence system or other programming to remotely perform methods of template generation, initialization, updating, therapy association, and/or selection in accordance with the present invention.

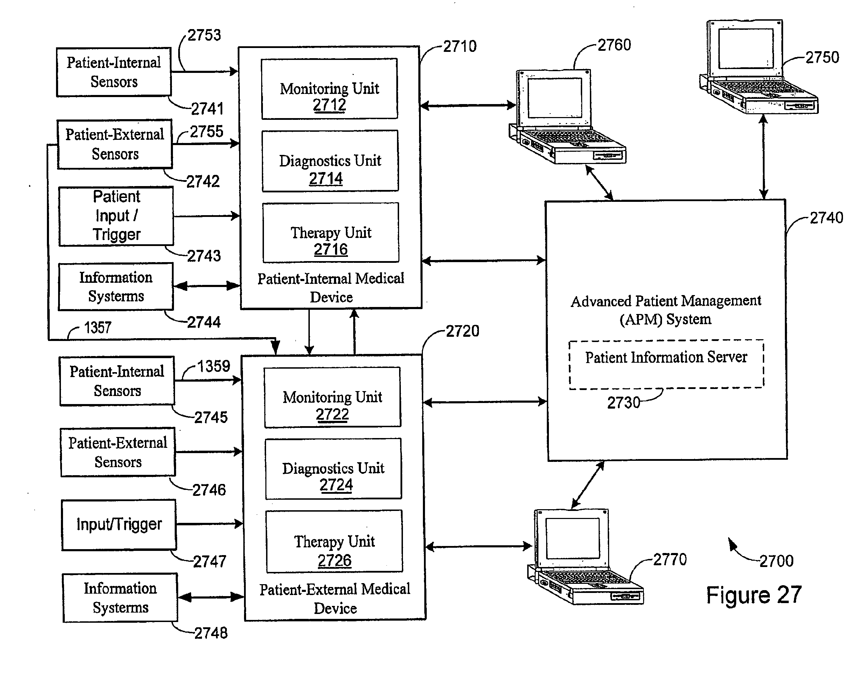

Referring now to Figure 27, a CRM of the present invention may be used within the structure of an APM system 2700. The APM system 2700 allows physicians and/or other clinicians to remotely and automatically monitor cardiac and respiratory functions, as well as other patient conditions. The APM system 2700 may also be used to provide information to the CRM for incorporation into templates, such as medication information or other patient information useful in accordance with the present invention. The APM system 2700 may also be used to select portions of cardiac waveforms for which templates are desired. The APM system 2700 may also be used to select or eliminate therapies associate with templates. In one example, a CRM implemented as a cardiac pacemaker, defibrillator, or resynchronization device may be equipped with various telecommunications and information technologies that enable real-time data collection, diagnosis, and treatment of the patient.

As is illustrated in Figure 27, the medical system 2700 may be used to implement template generation, template updating, template initialization, template selection, patient measuring, patient monitoring, patient diagnosis, patient therapy, therapy selection, and/or therapy elimination in accordance with embodiments of the invention. The medical system 2700 may include, for example, one or more patient-internal medical devices 2710, such as a CRM, and one or more patient-external medical devices 2720, such as a monitor or signal display device. Each of the patient-internal 2710 and patient-external 2720 medical devices may include one or more of a patient monitoring unit 2712, 2722, a diagnostics unit 2714, 2724, and/or a therapy unit 2716, 2726.

The patient-external medical device 2720 performs monitoring, and/or diagnosis and/or therapy functions external to the patient (i.e., not invasively implanted within the patient's body). The patient-external medical device 2720 may be positioned on the patient, near the patient, or in any location external to the patient.

The patient-internal and patient-external medical devices 2710, 2720 may be coupled to one or more sensors 2741, 2742, 2745, 2746, patient input / trigger devices 2743, 2747 and/or other information acquisition devices 2744, 2748. The sensors 2741, 2742, 2745, 2746, patient input/trigger devices 2743, 2747, and/or other information acquisition devices 2744, 2748 may be employed to detect conditions relevant to the monitoring, diagnostic, and/or therapeutic functions of the patient-internal and patient-external medical devices 2710,2720.

The medical devices 2710, 2720 may each be coupled to one or more patient-internal sensors 2741, 2745 that are fully or partially implantable within the patient. The medical devices 2710, 2720 may also be coupled to patient-external sensors positioned on, near, or in a remote location with respect to the patient. The patient-internal and patient-external sensors are used to sense conditions, such as physiological or environmental conditions, that affect the patient

The patient-internal sensors 2741 may be coupled to the patient-internal medical device 2710 through one or more internal leads 2753. Still referring to Figure 27, one or more patient-internal sensors 2741 may be equipped with transceiver circuitry to support wireless communications between the one or more patient-internal sensors 2741 and the patient-internal medical device 2710 and/or the patient-external medical device 2720. The patient-internal sensors 2745 may be coupled to the patient-external medical device 2720 through a wireless connection 2759, and/or using communications between the patient-internal medical device 2710 and the patient-external medical device 2720, or may be coupled using a wire or other communications channel.

The patient-external sensors 2742 may be coupled to the patient-internal medical device 2710 through one or more internal leads 2755. Patient-external sensors 2742 may communicate with the patient-internal medical device 2710 wirelessly. Patient-external sensors 2742 may be coupled to the patient-external medical device 2720 through one or more leads 2757 or through a wireless link.

In an embodiment of the present invention, the patient-external medical device 2720 includes a visual display configured to concurrently display non-electrophysiological signals and electrogram signals. For example, the display may present the information visually. The patient-external medical device 2720 may also, or alternately, provide signals to other components of the medical system 2700 for presentation to a clinician, whether local to the patient or remote to the patient.

Referring still to Figure 27, the medical devices 2710, 2720 may be connected to one or more information acquisition devices 2744, 2748, such as a database that stores information useful in connection with the monitoring, diagnostic, or therapy functions of the medical devices 2710, 2720. For example, one or more of the medical devices 2710, 2720 may be coupled through a network to a patient information server 2730.

The input/trigger devices 2743,2747 are used to allow the physician, clinician, and/or patient to manually trigger and/or transfer information to the medical devices 2710, 2720 and/or from the APM system 2740 and/or patient-external medical device 2720 back to the patient-internal device 2710. The input/trigger devices 2743,2747 may be particularly useful for inputting information concerning patient perceptions, such as a perceived cardiac event, how well the patient feels, and other information not automatically sensed or detected by the medical devices 2710, 2720. For example, the patient may trigger the input/trigger device 2743 upon perceiving a cardiac event. The trigger may then initiate the recording of cardiac signals and/or other sensor signals in the patient-internal device 2710. Later, a clinician may trigger the input/trigger device 2747, initiating the transfer of the recorded cardiac and/or other signals from the patient-internal device 2710 to the patient-external device 2720 for display and diagnosis.

In one embodiment, the patient-internal medical device 2710 and the patient-external medical device 2720 may communicate through a wireless link between the medical devices 2710, 2720. For example, the patient-internal and patient-external devices 2710, 2720 may be coupled through a short-range radio link, such as Bluetooth, IEEE 802.11, and/or a proprietary wireless protocol. The communications link may facilitate uni-directional or bi-directional comnnunication between the patient-internal 2710 and patient-external 2720 medical devices. Data and/or control signals may be transmitted between the patient-internal 2710 and patient-external 2720 medical devices to coordinate the functions of the medical devices 2710, 2720.

In another embodiment, patient data may be downloaded from one or more of the medical devices periodically or on command, and stored at the patient information server 2730. The physician and/or the patient may communicate with the medical devices and the patient information server 2730, for example, to acquire patient data or to initiate, terminate or modify recording and/or therapy.

The data stored on the patient information server 2730 may be accessible by the patient and the patient's physician through one or more terminals 2750, e.g., remote computers located in the patient's home or the physician's office. The patient information server 2730 may be used to communicate to one or more of the patient-internal and patient-external medical devices 2710, 2720 to provide remote control of the monitoring, diagnosis, and/or therapy functions of the medical devices 2710, 2720.

In one embodiment, the patient's physician may access patient data transmitted from the medical devices 2710, 2720 to the patient information server 2730. After evaluation of the patient data, the patient's physician may communicate with one or more of the patient-internal or patient-external devices 2710, 2720 through an APM system 2740 to initiate, terminate, or modify the monitoring, diagnostic, and/or therapy functions of the patient-internal and/or patient-external medical systems 2710, 2720.

In another embodiment, the patient-internal and patient-external medical devices 2710,2720 may not communicate directly, but may communicate indirectly through the APM system 2740. In this embodiment, the APM system 2740 may operate as an intermediary between two or more of the medical devices 2710, 2720. For example, data and/or control information may be transferred from one of the medical devices 2710, 2720 to the APM system 2740. The APM system 2740 may transfer the data and/or control information to another of the medical devices 2710, 2720.

In one embodiment, the APM system 2740 may communicate directly with the patient-internal and/or patient-external medical devices 2710, 2720. In another embodiment, the APM system 2740 may communicate with the patient-internal and/or patient-external medical devices 2710, 2720 through medical device programmers 2760, 2770 respectively associated with each medical device 2710, 2720. As was stated previously, the patient-internal medical device 2710 may take the form of an implantable PIMD.

Various modifications and additions can be made to the preferred embodiments discussed hereinabove without departing from the scope of the present invention. Accordingly, the scope of the present invention should not be limited by the particular embodiments described above, but should be defined only by the claims set forth below.

**Claims**

1. A medical system, comprising:

   a detector system (1370, 2712, 2722, 2741, 2742, 2745, 2746) configured to detect a cardiac waveform associated with a particular tachyarrhythmia episode;
   a processor (1390, 1395, 2714, 2724) coupled to the detector system and a cardiac therapy system, the processor configured to provide

   one or more templates representative of cardiac signals of one or more of a patient's previous tachyarrhythmia episodes, and
   patient information associated with the previous tachyarrhythmia episodes, each of the one or more templates associated with a therapy to treat the previous tachyarrhythmia episodes; and

   the cardiac therapy system (1365, 1375, 2716, 2726) configured to deliver a cardiac therapy to a patient, the cardiac therapy system configured to deliver therapy to treat the particular tachyarrhythmia episode based on whether the detected cardiac waveform corresponds to a particular template of the one or more templates and based on the patient information,
   **characterised in that** the patient information comprises whether the previous tachyarrhythmia episodes were sustained or non-sustained and the processor is configured to delay therapy for a period of time associated with the duration of at least one of the previous non-sustained tachyarrhythmia episodes if the particular tachyarrhythmia episode is of a non-sustained type.

2. The system of claim 1, wherein the patient information comprises a history of success of a previously delivered therapy.

3. The system of claim 1, wherein the patient information comprises patient activity information, acceleration information and/or respiration information.

4. The system of claim 1, wherein the patient information comprises neural activity information and/or pharmacological information.

5. The system of claim 1, wherein the patient information comprises hemodynamic information, blood pressure and/or blood oxygen information.

6. The system of claim 1, wherein the patient information comprises impedance information.

7. The system of claim 1, wherein the patient information comprises history of patient response to a particular therapy.

8. The system of claim 1, wherein the one or more templates comprise a plurality of ventricular tachyarrhythmia templates (VT).

9. The system of claim 1, wherein the one or more templates comprise a plurality of supraventricular tachyarrhythmia (SVT) templates.

**10.** The system of claim 1, wherein the patient information is measured at or near a time of occurrence of the tachyarrhythmia episode.

**11.** The system of claim 1, wherein the patient information includes a duration of the previous tachyarrhythmia episodes, and/or whether the previous tachyarrhythmia episodes were reclassified during an increased evaluation period.

**12.** The system of claim 1, wherein the patient information is used to arbitrate between two templates that correspond to the cardiac waveform.

**13.** The system of claim 1, wherein the patient information is used to select an SVT template.

**14.** The system of claim 1, wherein the patient information is used to select the cardiac therapy.

**Patentansprüche**

**1.** Medizinisches System, welches aufweist:

ein Detektorsystem (1370, 2712, 2722, 2741, 2742, 2745, 2746), das konfiguriert ist zum Erfassen einer Herzwellenform, die mit einer bestimmten Tachyarrhythmie-Episode assoziiert ist;
einen Prozessor (1390, 1395, 2714, 2724), der mit dem Detektorsystem und einem Herztherapiesystem gekoppelt ist, welcher Prozessor konfiguriert ist, vorzusehen:

eine oder mehrere Schablonen, die für Herzsignale von einer oder mehreren vorhergehenden Tachyarrhythmie-Episoden eines Patients repräsentativ sind, und
Patienteninformationen, die mit den vorhergehenden Tachyarrhythmie-Episoden assoziiert sind, wobei jede von der einen oder den mehreren Schablonen mit einer Therapie zum Behandeln der vorhergehenden Tachyarrhythmie-Episoden assoziiert sind; und

das Herztherapiesystem (1365, 1375, 2716, 2726) konfiguriert ist, einem Patienten eine Herztherapie zuzuführen, wobei das Herztherapiesystem konfiguriert ist, eine Therapie zum Behandeln der bestimmten Tachyarrhythmie-Episode auf der Grundlage dessen, ob die erfasste Herzwellenform einer bestimmten Schablone von der einen oder den mehreren Schablonen entspricht, und auf der Grundlage der Patienteninformationen zu liefern,
**dadurch gekennzeichnet, dass** die Patienteninformationen enthalten, ob die vorhergehenden Tachyarrhythmie-Episoden ausgedehnt oder nicht ausgedehnt waren, und der Prozessor konfiguriert ist, die Therapie um eine Zeitperiode zu verzögern, die mit der Dauer zumindest einer der vorhergehenden, nicht ausgedehnten Tachyarrhythmie-Episoden assoziiert ist, wenn die spezifische Tachyarrhythmie-Episode von einem nicht ausgedehnten Typ ist.

**2.** System nach Anspruch 1, bei dem die Patienteninformationen eine Historie des Erfolgs einer vorhergehend zugeführten Therapie enthalten.

**3.** System nach Anspruch 1, bei dem die Patienteninformationen Patientenaktivitätsinformationen, Beschleunigungsinformationen und/oder Atmungsinformationen enthalten.

**4.** System nach Anspruch 1, bei dem die Patienteninformationen Informationen über neurale Aktivität und/oder pharmakologische Informationen enthalten.

**5.** System nach Anspruch 1, bei dem die Patienteninformationen hämodynamische Informationen, Blutdruck- und/oder Blutsauerstoffinformationen enthalten.

**6.** System nach Anspruch 1, bei dem die Patienteninformationen Impedanzinformationen enthalten.

**7.** System nach Anspruch 1, bei dem die Patienteninformationen eine Historie des Patientenansprechens auf eine bestimmte Therapie enthalten.

**8.** System nach Anspruch 1, bei dem die eine oder die mehreren Schablonen mehrere ventrikuläre Tachyarrhythmie-Schablonen (VT) enthalten.

9. System nach Anspruch 1, bei dem die eine oder die mehreren Schablonen mehrere supraventrikuläre Tachyarrhythmie(SVT)-Schablonen aufweisen.

10. System nach Anspruch 1, bei dem die Patienteninformationen zu oder nahe einer Zeit des Auftretens der Tachyarrhythmie-Episode gemessen werden.

11. System nach Anspruch 1, bei dem die Patienteninformationen eine Dauer der vorhergehenden Tachyarrhythmie-Episoden enthalten und/oder, ob die vorhergehenden Tachyarrhythmie-Episoden während einer Periode erhöhter Auswertung neu klassifiziert wurden.

12. System nach Anspruch 1, bei dem die Patienteninformationen dazu verwendet werden, zwischen zwei Schablonen, die der Herzwellenform entsprechen, zu entscheiden.

13. System nach Anspruch 1, bei dem die Patienteninformationen dazu verwendet werden, eine SVT-Schablone auszuwählen.

14. System nach Anspruch 1, bei dem die Patienteninformationen dazu verwendet werden, die Herztherapie auszuwählen.


**Revendications**

1. Système médical, comprenant :

   un système de détecteur (1370, 2712, 2722, 2741, 2742, 2745, 2746) configuré pour détecter une forme d'onde cardiaque associée à un épisode de tachyarythmie particulier ;
   un processeur (1390, 1395, 2714, 2724) couplé au système de détecteur et à un système de thérapie cardiaque, le processeur étant configuré pour fournir :

      un ou plusieurs gabarits représentatifs de signaux cardiaques d'un ou de plusieurs épisodes de tachyarythmie antérieurs de patient, et
      une information de patient associée aux épisodes de tachyarythmie antérieurs, chacun des un ou plusieurs gabarits étant associé à une thérapie pour traiter les épisodes de tachyarythmie antérieurs ; et

   le système de thérapie cardiaque (1365, 1375, 2716, 2726) étant configuré pour délivrer une thérapie cardiaque à un patient, le système de thérapie cardiaque étant configuré pour délivrer une thérapie afin de traiter l'épisode de tachyarythmie particulier sur la base de si oui ou non la forme d'onde cardiaque détectée correspond à un gabarit particulier des un ou plusieurs gabarits et sur la base de l'information de patient, **caractérisé en ce que** l'information de patient comprend si les épisodes de tachyarythmie antérieurs ont été soutenus ou non soutenus et le processeur est configuré pour retarder la thérapie pendant une période temporelle associée à la durée d'au moins l'un des épisodes de tachyarythmie non soutenus antérieurs si l'épisode de tachyarythmie particulier est d'un type non soutenu.

2. Système selon la revendication 1, dans lequel l'information de patient comprend un historique de succès d'une thérapie délivrée antérieurement.

3. Système selon la revendication 1, dans lequel l'information de patient comprend une information d'activité de patient, une information d'accélération de patient et/ou une information de respiration de patient.

4. Système selon la revendication 1, dans lequel l'information de patient comprend une information d'activité neurale et/ou une information pharmacologique.

5. Système selon la revendication 1, dans lequel l'information de patient comprend une information hémodynamique, une pression sanguine et/ou une information d'oxygène.

6. Système selon la revendication 1, dans lequel l'information de patient comprend une information d'impédance.

**7.** Système selon la revendication 1, dans lequel l'information de patient comprend un historique de réponses de patient à une thérapie particulière.

**8.** Système selon la revendication 1, dans lequel les un ou plusieurs gabarits comprend/comprennent une pluralité de gabarits de tachyarythmie ventriculaire (VT).

**9.** Système selon la revendication 1, dans lequel les un ou plusieurs gabarits comprend/comprennent une pluralité de gabarits de tachyarythmie supraventriculaire (SVT).

**10.** Système selon la revendication 1, dans lequel l'information de patient est mesurée à l'instant de la survenue de l'épisode de tachyarythmie ou à proximité de cet instant.

**11.** Système selon la revendication 1, dans lequel l'information de patient inclut une durée des épisodes de tachyarythmie antérieurs, et/ou si oui ou non les épisodes de tachyarythmie antérieurs ont été classifiés pendant une période d'évaluation augmentée.

**12.** Système selon la revendication 1, dans lequel l'information de patient est utilisée pour réaliser un arbitrage entre deux gabarits qui correspondent à une forme d'onde cardiaque.

**13.** Système selon la revendication 1, dans lequel l'information de patient est utilisée pour sélectionner un gabarit SVT.

**14.** Système selon la revendication 1, dans lequel l'information de patient est utilisée pour sélectionner la thérapie cardiaque.

110

Detect Cardiac Beats of an
Arrhythmic Episode

120

Compare Morphology of Cardiac Beats
with Representative Beat Morphologies

130

Classify the Arrhythmic Episode as a
Particular Type of Arrhythmia

Figure 1A

```
                                                                    ┌─ 122
        ┌──────────────────────────────────────────┐
        │          Detect Cardiac Beats             │
        └──────────────────────────────────────────┘
                            │
                            │                        ┌─ 124
                            ▼
        ┌──────────────────────────────────────────┐
        │   Compare Morphology of Cardiac Beats     │◄──┐
    ┌──►│   with Representative Beat Morphology      │   │
    │   └──────────────────────────────────────────┘   │
    │                       │                           │
    │                       ▼           ┌─ 126          │
    │                  ╱────────────╲                   │
    │                 ╱ Cardiac Beats ╲                 │
    │                ╱  Consistent with ╲     no        │
    │                ╲ Representative Beat╱──────────┐  │
    │                 ╲   Morphology?    ╱           │  │
    │                  ╲────────────╱                │  │
    │                       │ yes                    │  │
    │                       ▼           ┌─ 128       │  │
    │   ┌──────────────────────────────────────────┐│  │
    │   │ Classify the Arrhythmic Episode as the    ││  │
    │   │ Particular Type of Arrhythmia             ││  │
    │   │ Associated with the Representative        ││  │
    │   │ Beat Morphology                           ││  │
    │   └──────────────────────────────────────────┘│  │
    │                       │                        │  │
    │                       ▼           ┌─ 130       │  │
    │                  ╱────────────╲                │  │
    │    yes          ╱ More Represent-╲             │  │
    └────────────────╱  ative Beat      ╲◄───────────┘  │
                     ╲  Morphologies to ╱                │
                      ╲   Compare?     ╱                 │
                       ╲────────────╱                    │
                            │ no                         │
                            ▼           ┌─ 132           │
        ┌──────────────────────────────────────────┐    │
        │   Classify the Arrhythmia Episode as an   │    │
        │          Unknown Arrhythmia               │    │
        └──────────────────────────────────────────┘    │
```

# Figure 1B

Sense an intrinsic electrical heart signal using an implantable medical device. ⌐ 210

Store a portion of the intrinsic electrical heart signal from a tachyarrhythmia episode. ⌐ 220

Does user input indicate that the tachyarrhythmia portion is indicative of SVT? ⌐ 230

No

Yes

Generate SVT template from the portion of the heart signal. ⌐ 240

Determine at least one degree of similarity between a second portion of the heart signal from a later tachyarrhythmia episode and at least one SVT template. ⌐ 250

Declare the second portion of the heart signal to represent a SVT episode if the at least one degree of similarity exceeds at least one threshold value. ⌐ 260

Figure 2A

Sense an intrinsic electrical heart signal using an implantable medical device. — 212

Store a tachyarrhythmia portion of the intrinsic electrical heart signal from a tachyarrhythmia episode. — 222

Determine at least one degree of similarity between the tachyarrhythmia portion of the heart signal and at least one SVT template. — 232

Does the at least one degree of similarity exceed at least one threshold value? — 242

Declare the portion of the heart signal to represent a SVT episode and suppress antitachyarrhythmia therapy. — 252

Yes

No

Declare the tachyarrhythmia portion of the heart signal to represent a VT episode and deliver antitachyarrhythmia therapy. — 262

Does user input indicate that tachyarrhythmia portion is indicative of SVT? — 272

Do not generate SVT template. — 292

No

Yes

Generate SVT template from the tachyarrhythmia portion of the heart signal. — 282

## Figure 2B

Sense an intrinsic electrical heart signal using an implantable medical device. — 205

Store portions of the intrinsic electrical heart signal from tachyarrhythmia episodes. — 215

Present to user graphical representation of portions of the intrinsic electrical heart signal from tachyarrhythmia episodes. — 225

Does arrhythmia represented by portion of signal require antitachyarrhythmia therapy? — 235

Yes

No

Receive input from user designating portion of intrinsic electrical heart signal that should be used to generate template. — 245

Generate template from the designated portion of the heart signal. — 255

Determine at least one degree of similarity between a second portion of the heart signal from a later tachyarrhythmia episode and at least one template. — 265

Does the at least one degree of similarity exceed at least one threshold value? — 275

No

Yes

Suppress antitachyarrhythmia therapy. — 285

Figure 2C

Figure 3

Figure 4

510 — Monitor a heart signal.

520 — Identify portions of the heart signal that are indicative of SVT.

530 — Store the portions of the heart signal that are indicative of SVT as SVT candidate templates.

540 — Discard the oldest SVT template and save a new template.

550 — Track the frequency of similarity of a portion of the heart signal from a tachyarrhythmia episode to one of a plurality of SVT templates.

560 — Discard the SVT template exhibiting the least frequent correlation with the heart signal and save a new template.

570 — Discard an SVT template with a heart rate similar to heart rates of other SVT templates.

580 — Discard a SVT template that is associated with a discontinued drug therapy regimen.

590 — Discard an SVT template that is most similar to other SVT templates.

## Figure 5

Figure 6

EP 2 380 626 B1

700

710

720

Processor

Determine at least one degree of similarity between a first portion of the heart signal from a tachyarrhythmia episode and at least one supraventricular tachyarrhythmia (SVT) template representative of a previous SVT episode.

740

Sensor circuitry

720

intrinsic heart signal

770

lead

Heart

antitachyarrhythmia therapy

Suppress ventricular antitachyarrhythmia therapy if the at least one degree of similarity exceeds at least one threshold value.

750

Memory circuit

760

Figure 7

EP 2 380 626 B1

## NSR Template

## Figure 8

## SVT

## Figure 9

Associate Cardiac Therapies with
Types of Arrhythmia

1040

Detect Cardiac Beats

1042

Compare Morphology of Cardiac Beats
with Representative Beat Morphologies

1044

Classify the Arrhythmic Episode as a
Particular Type of Arrhythmia

1046

Deliver a Therapy Associated with the
Particular Type of Arrhythmia

1048

Figure 10

EP 2 380 626 B1

Figure 11

41

Figure 12

Figure 13A

1350

Can
Electrode          1355

Template
Processor          1365

Pacing Therapy
Circuitry

1395

1360

Control System
1390

1370

Sensing Circuitry

Lead System/
Electrodes

1375

Shock Therapy
Circuitry

1380          1385          1386

Power Supply     Communications
Circuitry          Memory

1351

1392

Programmer          1394

Figure 13B

44

Figure 14

Figure 15

## RATE CHANNEL SIGNAL

Figure 16A

## SHOCK CHANNEL SIGNAL

Figure 16B

Figure 17

1810

Episode Detected

1820

Compare Episode To At Least One Template

1830

Features Match Template?

yes

1850

Previous Therapy Effective?

no

no

yes

1860

Deliver Previous Therapy

1870

Deliver Different And/Or More Aggressive Therapy

Create New Template

1840

Figure 18

1910

Establish Initial Template List
(Optional)

1920

Detect Ventricular Tachycardia

1930

Detect Features of Cardiac Waveform

1940

Compare Waveform Features to
Template

1950

Features Match
Template?

yes          no

1960

Use Template Information
For Therapy Selection

1955

More Templates to
Compare?          yes

no          1970

Form New VT Template

Figure 19

Figure 20

Figure 21

```
                        ┌─────────────────────────┐ ⟋2205
                        │   Detect Cardiac Beats   │
                        └─────────────────────────┘
                                     │
                                     ▼
                        ┌─────────────────────────┐ ⟋2210
                        │ Compare Morphology of    │◄────────────┐
                        │ Cardiac Beats with       │             │
                        │ Representative Beat      │             │
                        │ Morphology               │             │
                        └─────────────────────────┘             │
                                     │                      yes  │
                                     ▼                           │
                              ╱──────────────╲ ⟋2215      ╱──────────────╲ ⟋2230
                             ╱  Cardiac Beats  ╲         ╱ More           ╲
                            ╱ Consistent with   ╲  no   ╱ Representative   ╲
                            ╲ Representative     ╱─────►╲ Beat Morphologies ╱
                             ╲ Beat Morphology? ╱       ╲ to Compare?      ╱
                              ╲──────────────╱           ╲──────────────╱
                                     │ yes                      │ no
                                     ▼ ⟋2220                     ▼ ⟋2235
                        ┌─────────────────────────┐   ┌─────────────────────────┐
                        │ Classify the Cardiac     │   │ Classify the Cardiac     │
                        │ Beats as the Particular  │   │ Beats as the Unknown     │
                        │ Type of Arrhythmia       │   │ Arrhythmia               │
                        │ Associated with the      │   └─────────────────────────┘
                        │ Representative Beat       │               │
                        │ Morphology               │               ▼
                        └─────────────────────────┘            ╱────╲
                                     │ ⟋2225                   │  A  │
                                     ▼                          ╲────╱
                        ┌─────────────────────────┐
                        │ Deliver a Therapy        │
                        │ Associated with the      │
                        │ Particular Type of       │
                        │ Arrhythmia               │
                        └─────────────────────────┘
```

Figure 22A

Figure 22B

Detect Cardiac Beats — 2275

Compare Morphology of Cardiac Beats with
Representative Beat Morphology — 2277

Cardiac Beats
Consistent with Representative
Beat Morphology? — 2279

no →

More Representative
Beat Morphologies
to Compare? — 2281

yes →

no ↓

Classify the Cardiac Beats as
Unknown Arrhythmia — 2283

A

yes ↓

Classify the Arrhythmia as the
Particular Type of Arrhythmia
Associated with the Representative
Beat Morphology — 2280

Was Arrhythmia
Type Previously
Non-sustained? — 2282

no →

yes ↓

Increase Evaluation Interval — 2284

Arrhythmia Self-Terminated? — 2285

no →

Deliver Therapy — 2287

yes ↓

No Therapy Required — 2289

Figure 22C

2310

Provide Set of One or More Templates
Respectively Characterizing One or More
Types of Arrhythmia

2320

Associate Each Type of Arrhythmia with a
Therapy

2330

Detect Cardiac Beats and Compare with
Templates

2340

Correlated to a Template? — yes → **Deliver Previously Successful Therapy** — 2345

no

2360

Can Template be
Acquired? — no → B

yes

2370

Acquire Template Characterizing Type of
Arrhythmia

Therapy Successful? — 846

847 — Maintain Association of
Arrhythmia and Therapy — yes

no

2375

Deliver Next Programmed Therapy for
Characterizable Arrhythmia — no ← Retry Therapy? — 2348 — yes

2380

Therapy Successful? — yes → Associate Characterizable
ArrhythmiaType with Successful
Therapy — 2390

no

2392

Therapy Attempt
Limit Reached?

no

yes

C

Figure 23A

56

Figure 23B

Determine Fiducial Point of Rate Channel Signal — 2405

Align Shock Channel Signal with Template Using Fiducial Points — 2410

Determine Features of Aligned Shock Channel Signal — 2515

Calculate Feature Correlation Coefficient — 2420

FCC > X? — 2425

Yes

No

Cardiac Beat Correlated to Template — 2435

Cardiac Beat Uncorrelated to Template — 2430

# Figure 24

Figure 25

Figure 26

Figure 27

EP 2 380 626 B1

**EP 2 380 626 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20040167579 A
- US 20040111120 A
- US 20040171959 A
- US 4880005 A